# EUROPEAN PATENT APPLICATION

(11) **EP 1 347 301 A1**
(43) Date of publication of application: **24.09.2003**
(21) Application number: 01998828.6
(22) Date of filing: 30.11.2001
(51) Int. Cl.: G01N 33/543

(54) **METHOD FOR ULTRA-RAPID AND ULTRA-SENSITIVE MEASUREMENT**

(30) Priority: 01.12.2000 JP 2000367434; 15.12.2000 JP 2000381826; 28.12.2000 JP 2000400853; 10.05.2001 JP 2001140417; 05.06.2001 JP 2001170186; 22.06.2001 JP 2001189419; 05.07.2001 JP 2001204964; 25.07.2001 JP 2001224350
(71) Applicant: International Reagents Corporation, Kobe-shi Hyogo 651-2271 (JP)
(72) Inventor: ISHIKAWA, Eiji, Kobe-shi, Hyogo 651-2 (JP); ODAWARA, Takuya, Kobe-shi, Hyogo 651-2 (JP); KAJITA, Tadahiro, Kobe-shi, Hyogo 651-2 (JP)
(74) Representative: Krauss, Jan
(86) International application number: JP0110481
(87) International publication number: WO02044726

(57) **Abstract**

An improved (ultra-rapid, ultra sensitive and low cost) non-competitive complex-transfer assay method for analytes (substances to be assayed) which comprises the following three steps.
Step A: a step in which the complex(es) of an analyte with its specifically binding substance(s) (including modified or labeled ones) is formed on a solid phase (a first solid phase);
Step B: a step in which the complex(es) formed on the first solid phase in the step A is eluted and transferred to another solid phase (a second solid phase); and
Step C: a step in which the complex(es) transferred to the second solid phase in the step B is measured.

## Description

The application claims priority from Japan Patent Application No.2000-367434, NO.2000-381826, No.2000-400853, No.2001-140417, No.2001-170186, No.2001-189419, No.2001-204964, N0.2001-224350 which are incorporated herein by reference.

### Technical Field of the Invention

The present invention relates to an art which is utilized in many fields such as human clinical tests, veterinary tests and food hygiene tests. Particularly, it relates to a novel solid-phase assay method in which an analyte (a substance to be assayed) is measured in an ultra-rapid and ultra-sensitive manner using a substance(s) which specifically binds to the analyte (specifically binding substance)and a solid phase and, more particularly, it relates to a novel ultra-rapid and ultra-sensitive assay method in which the complex(es) of an analyte with its specifically-binding substance(s) (including modified or labeled ones) is formed on a solid phase (a first solid phase), then the complex(es) is eluted and transferred to another solid phase (a second solid phase) and finally the complex(es) on the second solid phase is measured by which the analyte is measured(hereinafter, described as non-competitive complex-transfer assay method), to an assay kit containing at least one solid phase and/or reagent for the said assay method and to an assay system containing solid phase, reagent and automated software for the said assay method.

### Background of the Invention

In many fields including human clinical tests, veterinary tests, food tests, etc., an assay method using a substance(s) which specifically binds to an analyte (specifically binding substance) and a solid phase has been widely used already. For example, there has been widely used an assay method in which the complex(es) of an analyte such as antigens, antibodies, DNAs and physiologically acting substances with its specifically-binding substance(s) such as antibodies, antigens, DNA fragments hybridizing to the DNA to be assayed and receptors, respectively, is formed on a solid phase and the complex(es) on the solid phase is measured.

A method for improving the sensitivity of the assay method described above using a specifically-binding substance(s) and a solid phase has also been developed (E. Ishikawa, Ultrasensitive and Rapid Enzyme Immunoassay, Laboratory Techniques in Biochemistry and Molecular Biology, vol. 27, S. Pillai, P. C. van der Vliet, eds., Elsevier, Amsterdam, pp. 79-191, 1999). In the improved method, the complex(es) of an analyte with its specifically binding substance(s) is formed on a solid phase (a first solid phase), the complex(es) is eluted from the first solid phase and transferred to another solid phase (a second solid phase) and the complex(es) on the second solid phase is measured by which the analyte is measured (hereinafter, described as non-competitive complex-transfer assay method).

However, any method has various disadvantages. Firstly, a long time is needed for trapping an analyte or the complex(es) on a solid phase. However, there has been no attempt at all to shorten the incubation time from the start of contact of reaction solution containing an analyte or the complex(es) with a solid phase which is to trap them until the start of separation of the both to less than 5 minutes for rapid assay of the analyte with keeping high trapping rate of the analyte or the complex(es) on the solid phase for sensitive assay of the analyte particularly in the non-competitive complex-transfer assay method. Far from this, there has not been even any report, patent(including that during application) and example using the incubation time merely shortened to less than 5 minutes regardless of the trapping rate. Secondly, a long time is needed for binding a specifically-binding substance(s) to an analyte. There has been no attempt to shorten the incubation time from the start of mixing the both until the start of separation of the complex(es) of the both from the specifically-binding substance(s) to less than 5 minutes for rapid assay of the analyte with keeping high binding rate of the both for ' sensitive assay of the analyte particularly in the non-competitive complex-transfer assay method. Far from this, there has not been even any report, patent (including that during application) and example using the incubation time merely shortened to less than 5 minutes regardless of the binding rate. Thirdly, in a solid-phase assay method including the steps of forming a complex(es) of an analyte with its specifically binding substance on a solid phase and eluting the complex(es) from the solid phase,a long time is needed for the elution. Especially in the non-competitive complex-transfer assay method described above, a long time is needed for eluting the complex(es) from the first solid phase. Although there is a patent application (JP-A-6-82453) claiming that elution of the complex(es) from the solid phase has been accomplished within ten minutes or within one minute, there has been no report, patent (including that during application) and example using the elution time shortened for rapid assay of an analyte with keeping high elution rate of the complex(es) for sensitive assay of an analyte. Fourthly, the number of times for washing a solid phase is many and a long time is needed for washing. Especially in the non-competitive complex-transfer assay method, there has been no report, patent (including that during application) and example using the number of times for washing the solid phases minimized with keeping low non-specific signals for sensitive assay of an analyte. Fifthly, increase in the number of times for washing the solid phase(s) is disadvantageously one of the causes for increase in total volumes of washing and waste solutions and consequently for increase in the size of vessels for the both resulting in a large size of automated assay apparatus. Sixthly, although luminescent assay method has been used due to its high sensitivity, there is a disadvantage that material, shape, size, quantity, etc. of the solid phase that can be used are limited. For example, the amount or concentration of magnetic fine particles that can be used is markedly limited due to quenching of luminescence. Seventhly, use of different types of solid phase as many as different analytes to be tested in a single automated apparatus is one of the causes for increasing the size of autoamted apparatus as well as the cost. There has been no report, patent (including that during application) and example overcoming this disadvantage particularly in the non-competitive complex-transfer assay method. Eighthly, the time for separation of serum or plasma from whole blood is a great factor to hamper a rapid utilization of assay results even after overcoming the disadvantages described above.

Thus, the present invention comprises the followings.
1. A non-competitive complex-transfer assay method for measuring analytes comprising the following three steps A, B and C using at least one of the methods described in the following (1) to (7).
   Step A: a step in which the complex(es)of an analyte( a substance to be assayed) with a substance(s) (including modified or labeled ones) which binds specifically to the analyte (hereinafter described as specifically binding substance) is formed on a solid phase (a first solid phase).
   Step B: a step in which the complex(es) formed on the first solid phase in the step A is eluted and transferred to another solid phase (a second solid phase).
   Step C: a step in which the complex(es) transferred to the second solid phase in the step B is measured.
   (1) a method using, in at least one of the steps A and B described above, at least one solid phase in a form of small or fine particles which has a substance(s) insolubilized for trapping an analyte or the complex(es) of the analyte with its specifically binding substance(s) (including labeled or modified ones) and has such a large surface area that the amount of the analyte or the complex(es) trapped on the solid phase by 3 minute incubation of the both is 60% or more of the maximum amount of the analyte or the complex(es) trapped on the solid phase by a sufficiently long incubation of the both which is defined as 100%.
   (2) a method using at least one concentration of a specifically binding substance (including labeled or modified ones )which is so high that the amount of the complex(es) formed of the specifically binding substance with the corresponding analyte by 3 minute incubation of the both is 60% or more of the maximum amount of the complex(es) formed by a sufficiently long incubation of the both which is defined as 100%.
   (3) a method using at least one bond between the complex(es) and the first solid phase which ensures that the amount of the complex(es) eluted from the first solid phase by 3 minute incubation in the eluent is 60% or more of the total amount of the complex(es) fixed on the first solid phase before elution which is defined as 100%.
   (4) a method in which washing of the first solid phase after the formation of the complex(es) of an analyte with its specifically binding substance(s)(including modified or labeled ones) in the step A and before starting elution of the complex(es) in the step B(hereinafter, referred to as washing of the first solid phase) is carried out once and washing of the second solid phase after trapping the complex(es) in the step B and before starting the step C (hereinafter, referred to as washing of the second solid phase)is carried out once; or a method using two washings of the first solid phase and one washing of the second solid phase or no washing of the second solid phase.
   (5) a method using a common first solid phase and a common second solid phase for different analytes regardless of time and place of the assay.
   (6) a method in which luminescence intensity of reagent solution for luminescent assay incubated with a substance(s) bound to a solid phase which produces a luminescent substance(s) in the presence of the reagent solution or regulates its production is measured after its separation from the solid phase.
   (7) a method in which the complex(es) is formed on the first solid phase in the presence of whole blood which has been diluted 100-fold or less in a part or all of the step A.
2. The non-competitive complex-transfer assay method described in the above 1 using a solid phase(s) in a form of small or fine particles described in the above 1(1) which are magnetic or magnetizable ones.
3. The non-competitive complex-transfer assay method described in the above 1 or 2 using a solid phase(s) in a form of small or fine particles described in the above 1(1) which have a mean diameter of 0.2 to 200 µm or equivalent ones.
4. The non-competitive complex-transfer assay method described in the above 1 or 2, using a solid phase(s) in a form of small or fine particles described in the above 1(1) which have a mean diameter of 0.5 to 20 µm or equivalent ones.
5. The non-competitive complex-transfer assay method described in any of the above 1 to 4,using a solid phase(s) in a form of small or fine particles described in the above 1(1) having a surface area expressed in cm², the value of which is 50- to 1,000-fold larger than the value of the volume expressed in mL of the reaction solution containing an analyte or the complex(es) to be incubated with the solid phase or equivalent ones.
6. The non-competitive complex-transfer assay method described in any of the above 1 to 5, using the incubation time of an analyte or the complex(es) with a solid phase for trapping them described in the above 1(1) which is 10 seconds to 3 minutes.
7. The non-competitive complex-transfer assay method described in any of the above 1 to 6, using at least one concentration of specifically binding substances described in the above 1(2) which is 2 to 100 pmol/mL.
8. The non-competitive complex-transfer assay method described in any of the above 1 to 7, using the incubation time of an analyte and its specifically binding substances described in the above 1(2) which is 10 seconds to 3 minutes.
9. The non-competitive complex-transfer assay method described in any of the above 1 to 8, using the elution time of the complex(es) from the first solid phase described in the above 1(3) which is 10 seconds to 3 minutes.
10. The non-competitive complex-transfer assay method described in any of the above 1 to 9, using a bond of antihapten antibody(ies) with a hapten (including a hapten derivative(s)) for at least one bond between the complex(es) and the solid phase described in the above 1(3) and using the hapten (including a hapten derivative(s)) for elution of the complex(es) from the solid phase.
11. The non-competitive complex-transfer assay method described in the above 10, using an anti-dinitrophenyl group antibody(ies) as the antihapten antibody and a compound containing dinitrophenyl group(s) as the hapten (including a hapten derivative(s)).
12. A monoclonal anti-dinitrophenyl group antibody, retaining such a property that the bond between the antibody and the compound containing dinitrophenyl group(s) described in the above 11 is dissociated to an extent of 60% or more within 3 minutes after addition of 1 to 3 mM dinitrophenyl-lysine.
13. The monoclonal antibody described in the above 12 produced by hybridoma cells of the international deposition number FERM BP-8341 (the deposition number FERM P-18079).
14. A hybridoma cell of the international deposition number PERM BP-8341 (the deposition number FERM P-18079).
15. The non-competitive complex-transfer assay method described in the above 10 or 11 using the monoclonal antibody described in the above 12 or 13.
16. The non-competitive complex-transfer assay method described in any of the above 1 to 11 and 15 using the total time for conducting the steps A and B excluding the time for washing the solid phases which is 2 minutes to 10 minutes in combination with at least the three methods described in the above 1(1), 1(2) and 1(3).
17. The non-competitive complex-transfer assay method described in any of the above 1 to 11, 15 and 16 using two or three washings of the first solid phase in a form of small or fine particles described in the above 1(1) and one or two washings of the second solid phase in a form of small or fine particles described in the above 1(1) on condition that the solid phase in a form of small or fine particles is washed by dispersing the particles in washing solution(hereinafter, referred to as dispersed washing).
18. The non-competitive complex-transfer assay method described in the above 17 substituting, for one of the two or three washings of the first solid phase, one washing in which accumulated particles are washed without dispersion (hereinafter, referred to as accumulated washing).
19. The non-competitive complex-transfer assay method described in the above 17 or 18 substituting one accumulated washing for one of the one or two washings of the second solid phase.
20. The non-competitive complex-transfer assay method described in any of the above 1 to 11 and 15 to 19, using a common first solid phase for different analytes described in the above 1(5) on which monoclonal antihapten antibody(ies) has been insolubilized.
21. The non-competitive complex-transfer assay method described in the above 20 using a monoclonal anti-2,4-dinitrophenyl group antibody or the antibody described in the above 12 or 13 as monoclonal antihapten antibody.
22. The non-competitive complex-transfer assay method described in any of the above 1 to 11 and 15 to 21 using a common second solid phase for different analytes described in the above 1(5) on which monoclonal antihapten antibody(ies) which is different from that insolubilized on the first solid phase has been insolubilized.
23. The non-competitive complex-transfer assay method described in any of the above 1 to 11 and 15 to 22 using a common second solid phase for different analytes described in the above 1(5) on which avidin or streptavidin has been insolubilized.
24. The non-competitive complex-transfer assay method described in any of the above 1 to 11 and 15 to 23 using hydrolase(s) as a substance(s) which is bound to a solid phase and produces a luminescent substance(s) in the presence of the reagent solution for luminescent assay described in the above 1(6).
25. The non-competitive complex-transfer assay method described in the above 24 using alkaline phosphatase as hydrolase.
26. The non-competitive complex-transfer assay method described in the above 24 using β -D-galactosidase as hydrolase.
27. The non-competitive complex-transfer assay method described in any of the above 24 to 26 using a reagent solution for luminescent assay containing a dioxetane derivatives) as a luminescent substrate.
28. An assay kit containing at least one solid phase and/or reagent for carrying out the non-competitive complex-transfer assay method described in any of the above 1 to 11 and 15 to 27.
29. An assay system containing at least one solid phase and/or reagent for carrying out the non-competitive complex-transfer assay method described in any of the above 1 to 11 and 15 to 27.

### Problems that the Invention is to Solve

The problems that the present invention is to solve are to achieve an ultra-rapid assay and ultra-high sensitivity and to lower the cost in an assay method in which the complex(es) of a substance to be assayed (analyte)with a substance(s) (including modified or labeled ones) which specifically binds to the analyte (specifically binding substance)is formed on a solid phase (a first solid phase), then the complex(es) is eluted from the first solid phase and transferred to another solid phase (a second solid phase) and finally the complex(es) on the second solid phase is measured by which the analyte is measured (hereinafter, referred to as non-competitive complex-transfer assay method).

### Means for Solving the Problems

The present inventors have succeeded in solving the problems and have achieved the present invention by combining the noncompetitive complex-transfer assay method with a method in which an analyte or the complex(es) is trapped on the solid phase more completely and more rapidly by using a solid phase(s) with larger surface areas, a method in which the complex(es) of an analyte with its specifically-binding substance(s) (including modified or labeled ones) is formed more completely and more rapidly by using the specifically binding substance(s) (including modified or labeled ones) of higher concentrations, a method using a bond between a solid phase and the complex(es) of an analyte with its specifically-binding substance(s) which ensures more rapid and more complete elution of the complex(es) from the solid phase, a method for shortening the time required for washing the solid phases and, at the same time, lowering the cost of the assay by minimizing the number of times for washing of the solid phases, a method for lowering the cost of the assay by minimizing the number of different solid phases used for different analytes and/or a method in which luminescent intensity of a reagent solution for luminescent assay incubated with a label substance for assay of the complex(es) bound to a solid phase is measured after removal of the solid phase.

### Mode for Carrying Out the Invention

The present invention will now be illustrated in detail below.

The present invention can be applied to an assay method in which the complex(es) of a substance to be assayed (analyte) with a substance(s) which specifically binds to the analyte (including modified or labeled ones) (specifically binding substance)is formed on a solid phase (a first solid phase), then the complex(es) is eluted and transferred to another solid phase (a second solid phase) and finally the complex(es) on the second solid phase is measured (hereinafter, referred to as non-competitive complex-transfer assay method).

The substances to be assayed (analytes) in the present invention are all the substances for which specifically binding substances are available. Examples of the analytes classified on the basis of the type of specifically binding substances are antigens, antibodies, receptors, ligands, lectins, sugar chain compounds, RNAs, DNAs, haptens, etc. Examples of the analytes classified on the basis of their functions include hormones, immunoglobulins, coagulation factors, enzymes, drugs, etc. In terms of substance name, they include serum albumins, macroglobulins, ferritin, α-fetoprotein, CEA, prostate-specific antigen (PSA), hepatitis B virus surface antigen (HBsAg), HIV-1p24, etc. These analytes are contained, in many cases, in blood, saliva, urine, snot, tear, feces, tissue extracts, cultured media, etc. The analytes may be any of natural substances, artificially synthesized substances, products by genetic recombination, etc. and are limited neither by their origins, existing states, etc. nor by exemplification.

The specifically binding substances in the present invention are the substances which specifically bind to the substances to be assayed (analytes). The examples of specifically binding substance to analyte are antibody to antigen, antigen to antibody, antihapten antibody to hapten, hapten to antihapten antibody, hybridizable DNA to DNA, avidin or streptavidin to biotin, biotin or biotinylated protein to avidin or streptavidin, hormone (such as insulin) to hormone receptor (such as insulin receptor), hormone receptor (such as insulin receptor) to hormone (such as insulin), corresponding sugar chain to lectin and corresponding lectin to sugar chain. Also included are their fragments or subunits with specifically binding ability and those modified or labeled, the examples of which are biotinylated antigen, biotinylated antibody, biotinylated Fab', enzyme-labeled antigen, enzyme-labeled antibody, enzyme-labeled Fab', haptenized antigen, haptenized antibody, haptenized Fab', biotinylated receptor, biotinylated hormone, biotinylated hormone receptor, enzyme-labeled hormone receptor, etc. However, the said substances are not limited to the above examples.

The present invention uses any solid phase that has been used in the conventional solid-phase assay methods and will be newly developed except those with large surface areas described below. They are not limited by their material, shape, size, etc. and include any of polystyrene balls, nylon balls, glass balls, inner surface of polystyrene test tubes, polystyrene microplates, latex particles, magnetic particles, etc in various sizes.

The complexes of analytes with their specifically binding substances (including modified or labeled ones) which are formed, fixed or trapped on or are eluted from the solid phases in the present invention are formed by specific bindings of the both. Since there is no limitation for the method of forming the complexes, they are formed by various methods or by various reaction orders. Thus, a solid phase, an analyte and its specifically binding substance may be successively subjected to reaction in various orders or two or three of them may be subjected to reaction simultaneously. The number of molecules of each component contained in the complex is not limited, but is one or more and less than several in many cases. The ratio of the number of molecules of the components in the complexes is not limited but is 1 to 10 in many cases.

Specifically binding substances in the complexes which are formed, fixed or trapped on or are eluted from the solid phases are not necessarily modified or labeled. For example, a complex comprising an analyte, its enzyme-labeled specifically binding substance and its specifically binding substance bound to a solid phase through physical adsorption can be eluted from the solid phase using a detergent(s). However, specifically binding substances are usually modified or labeled.

There are two purposes for modifying or labeling the specifically-binding substances. One is to fix or trap the complexes on the solid phases or elute them from the solid phases and the other is to measure the complexes.

Methods and types of substance used for the modification or labeling and the number of molecules of the substances introduced into the specifically binding substance molecules are not limited so far as the complexes can be fixed on, trapped on or eluted from the solid phases or can be measured rapidly with high sensitivity.

In order to fix or trap the complexes on the solid phases or elute the complexes from the solid phases, substances such as haptens, antihapten antibodies, charged substances, DNAs, substances containing thiol groups, substances containing thiopyridyl groups, ligands, receptors, lectins, sugar chains, biotin, avidin, streptavidin, etc. are used for the modification or labeling. The complexes containing the substances thus modified or labeled are fixed or trapped through hapten-antihapten antibody bond, ionic bond, DNA hybrid bond, disulfide bond, ligand-receptor bond, lectin-sugar chain bond, biotin-avidin bond, biotin-streptavidin bond, etc. on the solid phases on which antihapten antibodies, haptens, charged substances, DNAs, substances containing thiopyridyl groups, substances containing thiol groups, receptors, ligands, sugar chains, lectins, etc. have been insolubilized. The complexes are eluted from those solid phases by haptens, ions, high temperatures, reducing agents, ligands, saccharides, biotin, etc. Two or more of those bonds may be placed between the solid phases and the complexes which can be eluted by two or more substances or/and methods. However, biotin-avidin bond and biotin-streptavidin bond are rarely used for elution of the complexes.

In order to measure the complexes sensitively, substances which can be measured more rapidly and more sensitively are used as modification or label substances for assay (of the complexes). For example, enzymes, radioisotopes, fluorescent substances, luminescent substances, metals, etc. and, specifically, alkaline phosphatase, peroxidase, β-D-galactosidase, I¹³¹, I¹²⁵, fluorescein, aequorin, acridinium, europium and gold colloid are used as modification or label substances for assay (of the complexes).

The constitutions of the complexes are diverse, since specifically binding substances are modified or labeled in various ways and the complexes comprising both analytes and their specifically binding substances are formed and fixed in various manners. Their examples are (2,4-dinitrophenylated biotinylated specifically binding substance)-(analyte)-(specifically binding substance labeled with enzyme), (2,4-dinitrophenylated specifically binding substance)-(analyte)-(specifically binding substance labeled with enzyme),etc. 2,4-Dinitrophenyl groups and biotin may be replaced by charged substances, DNAs, thiol groups, etc. described above and the enzyme may be replaced by radioisotopes, fluorescent substances, luminescent substances, metals, etc. described above.

The eluting solutions (eluent)in the present invention are solutions for eluting the complexes comprising analytes and their specifically binding substances described above from the solid phases. Composition of the solutions varies depending on the state where the complexes are fixed on the solid phases. For the complexes fixed on the solid phases through physical adsorption, hapten-antihapten antibody bonds, ionic bonds and disulfide bonds, the eluting solutions contain detergents, haptens or their derivatives, charged substances and reducing agents,respectively. The eluting solutions with high temperatures are used for the complexes fixed through DNA hybrids. The eluting solutions are not limited to these examples.

Elution of the complexes in the present invention is carried out by various means. Usually, the eluting solutions and the solid phases on which the complexes have been fixed are contacted or mixed by shaking, stirring, etc. and, after a certain period of time,the both are separated. Various known means are also available for separation of the both. Examples include simple suction, centrifugal force, magnetic force, filtration, etc. for separation of solid phases in a form of particles. The methods used are not limited to the conventional ones.

The complexes comprising analytes and their specifically binding substances (including modified or labeled ones) eluted from the first solid phase can be trapped on the second solid phase by means of various bonds such as hapten-antihapten antibody bonds, DNA hybrid bonds, ionic bonds, biotin-avidin or streptavidin bonds, etc. which are different from those used for fixing them on the first solid phase. For this purpose, specifically binding substances have to be modified in advance of the assay by substances such as haptens, DNAs, charged substances, biotin, antihapten antibodies, avidin, streptavidin, etc. which are different from those used for fixing them on the first solid phase. On the second solid phase, antihapten antibodies, DNAs hybridizing to DNAs used for the modification, charged substances, avidin, streptavidin, biotin, etc. have to be insolubilized. The complexes can also be trapped on the second solid phase on which specifically binding substances being capable of binding to analytes have been insolubilized. For example, the complexes comprising antibodies, antigens and DNAs as analytes are trapped on the second solid phase on which anti-immunoglobulin antibodies, antibodies to the antigens and DNAs hybridizing to the DNAs,respectively,have been insolubilized.

The complexes comprising analytes and their specifically binding substances (including modified or labeled ones) transferred to the second solid phase can be measured by various methods. Labeled specifically binding substances for assay (of the complexes) are incorporated into the complexes in the step A or B in most cases, although possibly incorporated in the step C. The labels may be measured either without elution or after eluting a part or all of the complexes.

Although the complexes eluted from the first solid phase in the present invention are not dissociated in most cases, a part of them may be dissociated unless their trapping and assay are interfered with. For example, complexes of haptenized antigen-antibody-labeled antigen and haptenized antibody-antigen-labeled antibody may be dissociated to give antibody-labeled antigen and antigen-labeled antibody, since the partly dissociated complexes are trapped on the second solid phase on which anti-immunoglobulin antibodies and antibodies to the analyte antigen,respectively, have been insolubilized and can be measured.

The washing solutions for solid phases in the present invention may be either those used in the conventional solid phase assay methods or novel ones and are not limited by composition, pH, temperature, etc. For example, they may be various kinds of buffers or water which does or does not contain various kinds of proteins, detergents, animal serums, saccharides, lipids, etc.

The washing methods for solid phases in the present invention may be either those used in the conventional solid phase assay methods or novel ones and are not limited by operation, method, temperature, time, etc. For example, the whole surfaces of solid phases and the washing solutions are contacted or mixed by stirring, shaking, etc. and the washing solutions are separated from the solid phases by simple suction, centrifugation, magnetic force, etc. for latexes, magnetic beads etc. For the purpose of the present invention to provide an ultra-rapid and ultra-sensitive assay method by shortening the assay time, washing of the solid phases is preferably carried out as rapidly as possible.

The steps A, B and C of the non-competitive complex-transfer assay method used in the present invention can be carried out by various known methods (E. Ishikawa, Ultrasensitive and Rapid Enzyme Immunoassay, Laboratory Techniques in Biochemistry and Molecular Biology, Vol. 27, S. Pillai, P. C. van der Vliet eds., Elsevier, Amsterdam, pp. 79-191, 1999). However, the methods used in the present invention are not limited to the known ones and, at the same time, both steps A and B can be far more rapidly and easily carried out than the known ones, which is greatly different from the known assay methods.

The step A in the non-competitive complex-transfer assay method of the present invention comprises three different steps: a step in which a substance to be assayed (analyte) or the complex(es) of an analyte with its specifically binding substance(s) (including modified or labeled ones) is fixed on the first solid phase (step A-1), a step in which a specifically binding substance(s) (including modified or labeled ones)is bound to the corresponding analyte to form a complex (step A-2) and a step in which the first solid phase after the steps A-1 and A-2 is washed (step A-3). The step B also comprises three different steps: a step in which the complex (es) is eluted from the first solid phase after the step A (step B-1), a step in which the eluted complex (es) is trapped on the second solid phase (step B-2) and a step where the second solid phase after the step B-2 is washed (step B-3). The step C is a step in which the complex (es) transferred to the second solid phase is measured.

The present invention provides a non-competitive complex-transfer assay method for analytes which is less expensive, ultra-rapid and ultra-sensitive by a method in which analytes or the complexes are trapped on the first or/and second solid phases more completely and more rapidly using the solid phases with larger surface areas in the step A-1 or/and B-2 (Japanese Patent Application No. 2001-140417), a method in which the complexes are formed more completely and more rapidly using higher concentrations of specifically-binding substances (including modified or labeled ones) in the step A-2(Japanese Patent Application No. 2001-170186), a method in which the complexes are eluted from the first solid phase more completely and more rapidly using monoclonal antihapten antibodies with higher ability in the step B-1 (Japanese Patent Application 2000-400853), a method in which the number of times for washing the solid phases is minimized so that the steps A-3 and B-3 are carried out more rapidly and, at-the same time, the assay cost is lowered (Japanese Patent Application No. 2001-204964), a method in which a minimum number of different solid phases is used for different analytes, lowering the assay cost or/and a method in which label substances for assay of the complexes are measured with high sensitivity by measuring the luminescence intensity of reagent solutions for luminescent assay after separation from the solid phase in the step C(Japanese Patent Application No. 2001-189419). The present invention further provides an ultra-rapid and ultra sensitive non-competitive complex-transfer assay method for analytes contained in or mixed with whole blood in which interference of assay by whole blood is eliminated (Japanese Patent Application No. 2001·224350).

As hereunder, a method in which the step A-1 and/or B·2 are/is carried out more completely and more rapidly using solid phases of larger surface areas (Japanese Patent Application No. 2001-140417) will be illustrated in detail.

In order to carry out the non-competitive complex-transfer assay method with higher sensitivity and more rapidly, a substance to be assayed (analyte) or the complex(es) of an analyte with its specifically binding substance(s) (including modified or labeled ones) has to be trapped on the first solid phase or/and the second solid phase more completely and more rapidly. In the present invention, used are larger surface areas of the first solid phase or/and the second solid phase, on which a substance(s) for trapping the analyte or the complex(es) has been insolubilized, in comparison with the volume of reaction solutions containing the analyte or the complex(es), making the trap more rapid and more complete. Namely, the solid phase(s) used has such a large surface area(s) that the amount of the analyte or the complex(es) trapped by incubation of the reaction solution(s) with the solid phase(s) for as short a time as 3 minutes, 2 minutes or 1 minute is 60% or more, preferably 70% or more, more preferably 80% or more and most preferably 90% or more of the maximum amount trapped (defined as 100) by incubation for as sufficiently long a time as 10 minutes, 30 minutes or 60 minutes,providing ultra-rapid and ultra-sensitive assays.

Although the solid phases used in the present invention are not limited in terms of their material, shape, size, amount, etc., more rapid and more complete trapping of an analyte or the complex(es) using solid phases with large surface areas described above can be easily achieved using small or fine particles, whether magnetic or non-magnetic, which have smaller volumes relative to their surface areas. For example, a sphere solid phase of one-tenth in diameter has as small a volume as one-thousandth while the surface area is only one-hundredth (Table 1). For example, the diameter and the volume of a sphere with a surface area of 10 cm² are 1.785 cm and 2.98 mL, respectively, while the surface area and the volume of one hundred million spheres of 1/10,000 in diamater (1.785 um) are 10 cm² and 0.298 µL, respectively. When the specific gravity is 1.3, their weight is 0.387 mg. Although it is very difficult to uniformly contact the surface of a sphere having a diameter of 1.785 cm with 100 µL of reaction solution, it is easy to mix small particulate solid phase of 0.387 mg with 100 µL of reaction solution. Incidentally, many of commercially available magnetic beads have diameters of 0.90 to 1.8 µm. The diameters of the solid phases used in the present invention are 0.1 µm to 1 mm, preferably 0.2 to 200 µm and more preferably 0.5 to 20 µm assuming the solid phases to be spheres and there is no limitation for the shape so far as the solid phases are in similar sizes. Incidentally, the surface area and volume of 318 small spheres having a diameter of 1 mm are 10 cm² and 166 µL, respectively, and it is relatively easy to contact the spheres with 100 µL of reaction solution. There is no limitation for the material used either. The surface area used is that expressed in cm², the value of which is equal to the value of 20-fold to 5,000-fold larger volumes and preferably 50-fold to 1,000-fold larger volumes than the volume of the reaction solution expressed in mL. Thus, for 0.1 mL of the reaction solution, fine particle solid phases having surface areas of 2 cm² to 500 cm² and 5 cm² to 100 cm² in many cases are preferred. The amount of solid phases in a form of fine spherical particles having a diameter of 1 µm and a specific gravity of 1.3 used is 0.043 to 10.8 mg and 0.11 to 2.2 mg in many cases. For example, the ratio of the weight of magnetic beads used in the present invention to that used in a commercially available conventional sandwich assay kit for HBs Ag (Directions for Lumipulse II HBs Ag, Fuji Rebio; revised in March, 2000)(37.5µg/350µL) is at least 4 or more and 10 or more in many cases.

The incubation time of the reaction solution containing substances to be assayed (analytes) or the complexes of analytes with their specifically-binding substances (including modified or labeled ones) with the solid phase for trapping them is defined as the time from the start of mixing or contacting the reaction solution with the solid phase until the start of mixing or contacting the solid phase with the first washing solution after separation and removal of the reaction solution from the solid phase. In measuring the amount of the complexes trapped on the solid phase, mixing or contacting the reaction solution with the solid phase is started after formation of the complex in the reaction solution reaches equilibrium under the conditions of 25∼30°C, pH 6.5∼7.5 and ionic strength corresponding to 0.3M NaCl or less, and all the operations such as mixing, contacting, separation, removal, etc. shall be finished as rapidly as technically possible. However, it is acceptable that such conditions and operations for measuring the amount of analytes or the complexes trapped are different from those in the assay of analytes. In the assay of analytes, mixing or contacting of the reaction solution with the solid phase may be started before formation of the complex in the reaction solution reaches equilibrium.

The amount of analytes or the complexes trapped on the solid phase can be calculated from the amount of analytes remaining in the reaction solution or from the amount of analytes, their specifically binding substances (including modified or labeled ones) or label substances for assay bound to the solid phase.

In the assay of analytes in the present invention, at least one incubation time for trapping analytes or the complexes on the solid phase shall be 10 seconds to 3 minutes. Either a very rapid assay of analytes with a very large surface area of the solid phase or a relatively rapid assay of analytes with a relatively large surface area may be chosen for use.

The trapping rate of analytes or the complexes of analytes with their specifically-binding substances (including modified or labeled ones) on the solid phase described above is not correlated to any of specific amounts, amounts within specific ranges, specific concentrations and concentrations within specific ranges of analytes but is applicable to any amount or any concentration of analytes. It is usually meaningless to correlate the trapping rate to specific amounts, amounts within specific ranges, specific concentrations or concentrations within specific ranges of analytes. The reasons for this are as follows. In a typical non-competitive assay method used by the present invention, the signal value obtained in the step C is usually in linear relation to the amount or the concentration of analytes within the assay range of analytes adopted and consequently the trapping rate of analytes or the complexes on the solid phase is always constant within the assay range of analytes. Further, in the present invention, the assay range of antigens is 1 zmol to 1 pmol and 100 zmol to 0.01 pmol in many cases, varying with the type of antigens, and the amount or the concentration of antibodies is not expressed in gram(s) or mol(s)except in particular cases. Since, in many cases, the trapping rate of analytes or the complexes on the solid phase is calculated by measuring their amounts remaining in the reaction solution, it should be noted that no correct results can be obtained on measuring the amount or the concentration close to their detection limits.

As hereunder, a method using high concentrations of specifically-binding substances (including modified or labeled ones) to perform the step A·2 more completely and more rapidly (Japanese Patent Application No. 2001-170186) is illustrated in detail.

In order to carry out the non-competitive complex-transfer assay method more rapidly with higher sensitivity, analytes have to be bound to their specifically-binding substances (including modified or labeled ones) more rapidly and more completely. The present invention uses higher concentrations of specifically binding substances (including modified or labeled ones) so that their binding to analytes becomes more rapid and more complete. Namely, the concentration of specifically binding substances used is so high that the amount of the complexes of analytes {including analytes bound to their specifically binding substances (including modified or labeled ones) fixed on the solid phase} with their specifically binding substances (including modified or labeled ones) formed by incubation for as short a time as 3 minutes, 2 minutes or 1 minute is 60% or more, preferably 70% or more, more preferably 80% or more and most preferably 90% or more of the maximum amount formed(defined as 100%) by incubation for as sufficiently long a time as 10 minutes, 20 minutes, 60 minutes, etc.

The incubation time of analytes bound to the solid phase and the reaction solution containing their specifically binding substances (including modified or labeled ones) is defined as the time from the start of mixing or contacting the both until the start of mixing or contacting of the solid phase with washing solution after separation and removal of the reaction solution from the solid phase.

The incubation time for analytes(including analytes bound to their specifically binding substances or to those modified or labeled) in solution with their specifically binding substances (including modified or labeled ones) in solution is defined as the time from the start of mixing the both followed by incubation until the start of mixing or contacting washing solution with the solid phase for trapping the complexes of the both after separation and removal of the mixture of the both after incubation. In measuring the amount of the complexes of the both formed, the solid phase for trapping the complexes of the both has a surface area expressed in cm², the value of which is equal to the value of about 250-fold to about 1,500-fold larger volumes than that of the reaction solution expressed in mL on condition that 90% or more of the maximum amount of the complexes of the both formed are trapped by 0.5 to 1 minute incubation. The incubation time with the solid phase for trapping the complexes of the both is defined as the time from the start of mixing or contacting the solid phase with the mixture of the both until the start of mixing or contacting the solid phase with washing solution after separation and removal of the mixture of the both. For example, for 0.1 mL of the reaction solution, magnetic beads of about 1 µm diameter having a surface area of 25∼150 cm² are appropriate. The maximum amount of the complexes of the both formed is defined as the amount of the complexes formed and trapped on the solid phase for trapping the complexes of the both by incubation for as sufficiently long a time as 10 minutes or 60 minutes after the formation of the complexes of the both reaches equilibrium following incubation of the both for as sufficiently long a time as 10 minitues or 60 minutes.

The amount of the complexes of the both formed is measured under the condition of 25-30°C, pH 6.5∼7.5 and ionic strength corresponding to 0.3M NaCl or less and the operations such as mixing, contacting, separation, removal, etc. of the reaction solution, the solid phase, etc. shall be finished as rapidly as technically possible. The amount of the complexes of the both formed can be measured by estimating analytes or their specifically binding substances (including modified or labeled ones) bound to the solid phase.

In the assay of analytes according to the present invention, the concentration of their specifically binding substances (including modified and labeled ones) used for the formation of the complexes with analytes is 1 pmol/mL to 500 pmol/mL and 2 pmol/mL to 100 pmol/mL in many cases. At least one incubation time of analytes(including analytes bound to the solid phase) with their specifically binding substances (including modified or labeled ones) is 10 seconds to 3 minutes. Either a very rapid assay of analytes with a very high concentration of their specifically binding substances (including modified or labeled ones) or a relatively rapid assay with a relatively high concentration may be chosen for use.

For measuring the amount of the complexes of the both formed, operations, methods and rapidity for mixing, contacting, separation, removal, etc. of reaction solutions, solid phases, etc. reaction conditions, surface areas of solid phases, etc. are not necessarily the same as those for the assay of analytes. In the assay of analytes, it is not necessary to add a solid phase for trapping the complexes of the both after the formation of the complexes of analytes in solution with their specifically binding substances (including modified or labeled ones) reaches equilibrium.

As hereunder, a method in which the complex is more completely and more rapidly eluted from the first solid phase in the step B-1 (Japanese Patent Application No. 2000-400853) is illustrated in detail.

In order to carry out the non-competitive complex-transfer assay method more rapidly with higher sensitivity, the complexes have to be eluted from the first solid phase more rapidly and more completely. Although the complexes may be partially dissociated into constituting components during elution, not hampering the assay in some cases, the complexes formed and fixed on the first solid phase of analytes with their specifically binding substances (including modified or labeled ones) have to be eluted more rapidly and more completely without dissociation for more rapid and more highly sensitive non-completive complex-transfer assay in many cases.

In the present invention, at least one bond between the first solid phase and the complexes used ensures that the amount of the complexes eluted from the solid phase by incubation for as short a time as 3 minutes, 2 minute or 1 minute is 60% or more, preferably 70% or more, more preferably 80% or more and most preferably 90% or more of the total amount of the complex on the first solid phase before elution (defined as 100%).

Speed and/or degree of elution of the complexes from the solid phase depend(s) upon the property of the bond used for fixing of the complexes on the solid phase. For fixing the complexes on the solid phase, the present invention uses bonds which have uniform properties and ensure more rapid and more complete elution of the complexes. Since monoclonal antibody has a uniform property and a uniform binding ability to one epitope, bonds of monoclonal antibodies with epitopes have uniform properties. Among them, a bond of monoclonal anti-hapten antibody with hapten can be dissociated with excess of hapten or its derivatives and, therefore, is suitable as a bond used for placing between the complexes and the solid phase and for fixing the complexes on the solid phase in an assay method comprising the steps of formation and fixation of the complexes of analytes with their specifically binding substances on the solid phase and elution of the complexes from the solid phase. A bond of monoclonal anti-dinitrophenyl group (hereinafter, referred to as DNP) antibody with a dinitrophenyl compound is particularly suitable. As hereunder, preparation and utilization of monoclonal anti-DNP antibody is illustrated in detail as a specific example, although the present invention is not limited to this example.

### (Method for preparation of monoclonal antibody)

As a sensitizing antigen, a DNP derivative dissolved or suspended in an appropriate buffer such as phosphate buffer (PBS) is used. An antigen solution as immunogen usually has a concentration of about 50∼ about 500 µg/mL. In order to enhance the antigenicity, albumin; keyhole limpet hemocyanin (KLH), etc. are preferably chosen as a carrier protein. Examples of animals immunized and sensitized with the antigen are mouse, rat, horse, goat and rabbit, etc. Mouse is preferable and BALB/c mouse is more preferable.

In addition, in order to enhance the response of animals to be immunized to the antigen, it is possible to administer the antigen solution mixed with an adjuvant. Examples of adjuvants used in the present invention are Freund's complete adjuvant (FCA), Freund'S incomplete adjuvant (FIA), RIBI (MPL), RIBI (TDM), RIBI (MPL + TDM), *Bordetella pertussis* vaccine, muramyl dipeptide (MDP), aluminum adjuvant (ALUM) and their combinations. A combination of FCA in the first immunization and FIA or Ribi adjuvant.in the additional immunization is particularly preferred.

In the immunization method, injecting site, schedule, etc. may be appropriately changed depending on the type of antigen used with or without adjuvant, etc. For example, with mouse as an animal to be immunized, 0.05∼1 mL of antigen solution (10∼200 µg of immunogen) mixed with adjuvant is injected intraperitoneally, subcutaneously, intramuscularly or intravenously (in the tail) followed by one to four additional immunizations every 4∼21 days after the initial immunization and the final immunization 1∼4 weeks later. The antigen solution without adjuvant may be intraperitoneally injected in larger amounts. Antibody titer is checked using blood collected 5∼6 days after the additional immunization by the conventional method according to the antibody titer assay described below. Cells producing antibodies are obtained 3∼5 days after the final immunization by separation of spleen cells from the immunized animal.

Myeloma cells used are those derived from mouse, rat, human being, etc. Examples are mouse myeloma P3X63-Ag8, P3X63-Ag8-U1, P3NS1-Ag4, SP2/o-14 and P3X63-Ag8.653 etc. Antibody-producing cells and myeloma cells of identical species and, particularly, identifical strain are preferable. Myeloma can be stored by cryopreservation or maintained by subcultivation in a conventional medium containing serum of horse, rabbit or fetal calf. For cell fusion, cells at logarithmic phase are preferably used.Hybridoma cells are produced by fusion of antibody-producing cells and myeloma cells with methods using, for example, polyethylene glycol (PEG), Sendai virus, an electric fusion apparatus, etc. In a PEG method, for example, spleen cells and myeloma cells are suspended in a ratio of 1∼10:1 and preferably 5∼10:1 in a buffer or a medium containing 30∼ 60% PEG with an average molecular weight of 1,000∼6,000 and are allowed to react at 25∼ 37°C at pH 6∼8 for 30 seconds to 3 minutes. After the reaction, the cells are re-suspended in a medium devoid of PEG and cultured by planting in microtiter plates.

Hybridoma cells are selected by culture of the cells after fusion in a selection medium, usually, a hypoxanthine-aminopterin-thymidine (HAT) medium, in which parent cells are killed and only the hybridoma cells can proliferate. Selection of the hybridoma cells is started by exchanging a part, preferably, about a half of the medium for a selection medium usually 1∼7 day(s) after fusion and is continued by repeating medium exchange in the same manner every 2∼3 days. The wells in which the colonies are grown are confirmed under a microscope.

In order to check whether the growing hybridoma cells produce the desired antibody, the supernatant of the medium collected is subjected to an antibody assay. The antibody titer can be measured, for example, by adding the supernatant to hapten or haptenized protein insolublized followed by reaction with a secondary antibody (anti-globulin, anti-IgG, anti-IgM antibodies, etc.) labeled with fluorescent substances, enzymes, RIs, etc.

Further, a single clone is separated by a limiting dilution method, a soft agar method, a method using a fluorescence-activated cell sorter, etc. In a limiting dilution method, for example, it is possible to isolate hybridoma cell clones producing the desired monoclonal antibodies by culturing those diluted with the medium down to about 1 cell/well.

It is possible to select hybridoma cells for obtaining antibodies to meet the purpose of the present invention which have high ability in dissociation of the immune bonds between the antibodies and DNP compounds with DNP derivatives. In a method for this selection, for example, monoclonal anti-DNP antibodies showing high elution rates of immune complexes are selected by comparing signals obtained before and after elution with DNP derivatives to remove the immune complex of the antibodies and the labeled secondary antibodies following the addition of the supernatant of the medium to insolubilized DNP-BSA and subsequent reaction with a secondary antibody (such as anti-globulin, anti-IgG, anti-IgM antibodies, etc.) labeled with fluorescent substances, enzymes, RIs, etc. In order to obtain antibodies having high ability in the dissociation with DNP derivatives, for example, hybridoma cells showing high dissociation rates may be selected by comparing signals obtained with and without dissociation using a short dissociation time with DNP derivatives, DNP derivatives diluted with phosphate buffer such as PBS, etc.

Antibody-producing hybridoma cells thus obtained can be stored in the presence of a cryoprotectant such as about 10% (v/v) dimethyl sulfoxide (DMSO), glycerol, etc. at -70∼-196°C, for about half an year or longer time, even semipermanently. The cells are rapidly thawed in a constant-temperature water bath at about 37°C when needed and used desirably after well washing to eliminate residual cytotoxicity of the cryoprotectant.

The monoclonal antibodies obtained by the method described above in the present invention are specifically derived, for example, from mouse, belonging to an IgG class and are named DNP-649, DNP-1321, DNP-1402 (FERM P-18079), I)NP-1623 and DNP- 1753.

For examination of the class and subclass of antibodies produced by the hybridoma cells, antibodies secreted into the supernatant by the hybridoma cells cultured under the conventional condition are subjected to analysis using a commercially available kit for test of the class and subclass of the antibody.

The monoclonal antibodies may be prepared appropriately selectively either from ascites of mouse or by cell culture depending upon the amount required, property of the hybridoma cells, etc. Antibodies can be obtained in as high a concentration as several mg/mL from the ascites of hybridoma cells which can grow in the peritoneal cavity of mouse. Antibodies from hybridoma cells which can not grow *in vivo* are obtained from the supernatant of the cultured medium. Although the production of antibody by cell culture is lower in amount than that in vivo, it has an advantage of less contamination by immunoglobulins and other substances present in the peritoneal cavity, making the purification easy.

For preparation of monoclonal antibodies from the peritoneal cavity of mouse, collected is ascites accumulated 1∼3 weeks after transplantation of hybridoma cells (about 10⁶ or more) into the peritoneal cavity of BALB/c mouse to which a substance having an immunosuppressive action such as pristane (2,6,10,14-tetramethylpentadecane) has been administered. For hybridoma cells of different species (such as mouse and rat), use of nude mouse or irradiated mouse is preferred.

On the other hand, for preparation of antibodies from the supernatant of cell culture, the supernatant containing antibodies is obtained from the culture medium of hybridoma cells, for example, by a high-density culture method or a spinner flask culture method besides a stationary culture for maintenance of cells. Serum containing other antibodies, albumin, etc. and causing inconvenience in purification of monoclonal antibodies in many cases is added desirably in small amounts to the culture medium.

Monoclonal antibody can be easily purified from ascites and supernatant of the culture medium by conventional known methods such as fractionation with ammonium sulfate, sodium sulfate, polyethylene glycol and ethanol, DEAE ion-exchange chromatography, gel filtration, etc.

Anti-DNP monoclonal IgG from mouse for the present invention can be purified by affinity chromatography using protein A or anti-mouse immunoglobulin antibody-insolubilized carriers.

### (Embodiment of measurement of analytes)

The basic principle of assay using this antibody includes, for example, the following steps: a step for formation and fixation of a complex comprising the first two or all the three of an analyte, its specifically-binding substance having DNP groups and labeled specifically-binding substance on the surface of solid phase on which anti-DNP antibody has been insolubilized, a step for elution of the complex from the solid phase and a step for measurement of the complex trapped on another solid phase from the eluate. In another method, a complex comprising an analyte and its labeled specifically-binding substance having DNP group is formed, fixed, eluted and measured after trapping on another solid phase.

For the substances having DNP groups in the present invention, there is no particular limitation so far as DNP groups are bound to them to react with anti-DNP monoclonal antibodies. Examples are immunoglobulins, receptors, proteins, peptides, DNAs, etc. modified with DNP.

The assay in accordance with the present invention will be illustrated later in detail. An example will be carried out as follows, although the present invention is not limited to this.

Anti-DNP antibody insolublized on a microplate is allowed to react with an anti-analyte antibody modified by DNP, further with an analyte and then with labeled anti-analyte antibody. The immune complex comprising DNP-modified anti-analyte antibody, an analyte and labeled anti- (analyte) antibody is eluted from a microplate using εN-2, 4-DNP-L-lysine. Label of the immune complex trapped on another solid phase from the eluate is measured without dissociation from the solid phase or after its partial or total dissociation, by which the analyte is measured.

Examples of DNP include 2,4-DNP, 2,5-DNP and 2,6-DNP and 2,4-DNP is preferably used.

It is possible to select the monoclonal antihapten antibody so that the amount of the complex (es) comprising an analyte with its specifically binding substance(s) (including modified or labeled ones) eluted from the solid phase by incubation for as short a time as 3 minutes, 2 minutes, 1 minute or 0.5 minute is 60% or more, preferably 70% or more, more preferably 80% or more and most preferably 90% or more of the total amount (defined as 100%) of the complex(es) on the solid phase before elution. As a result, it is now possible to provide a more rapid and more highly sensitive assay method. The elution time of the complex from the solid phase is defined as the time from the start of mixing or contacting of the solid phase with the eluent until the start of mixing or contacting of the solid phase with washing solution after separation from the eluate. The elution rate of the complex from the solid phase is measured at 25∼30°C, at pH 6.5∼7.0 with ionic strength corresponding to 0.3M NaCl or less and all of the elution operations shall be carried out as rapidly as technically possible. It is acceptable that conditions, operations, etc. of the elution are different for measurement of the elution rate of the complex from the solid phase and for the assay of analytes. The elution time of the complex from the solid phase in the assay of analytes shall be from 10 seconds to 3 minutes. The eluent (solution for elution) described above is a solution having a composition which makes it possible to elute the complex from the solid phase. For example, for the complex fixed on the solid phase through the bond of anti-DNP antibody with DNP derivatives, a solution containing various kinds of DNP derivatives in various concentrations may be used as an eluent.

In order to elute the complex containing a dinitrophenylated specifically binding substance from the solid phase on which anti-DNP antibody has been insolubilized, DNP derivatives are used. Examples of DNP derivatives used are DNP-amino acids such as DNP-glycine, DNP-alanine, DNP-β-alanine and, particularly, εN-2, 4-DNP-L-lysine, although not limited to these derivatives. The concentration of DNP derivatives used is 1 mM to 5 mM in many cases, although not limited to those concentrations. Elution temperature is from room temperature to 37°C in many cases, although not limited to these.

AS hereunder, a method for rapid performance of the steps A-3 and B-3 by minimizing the number of times for washing the solid phases is illustrated (Japanese Patent Application No. 2001-204964).

In order to carry out the non-competitive complex-transfer assay method more rapidly and with higher sensitivity, labeled specifically binding substances hampering the correct measurement of the complexes in the step C have to be eliminated more rapidly and more completely by washing the first solid phase before eluting the complexes after their formation, that is, the first solid phase contacted with labeled specifically binding substances (antibodies, antigens, DNAs, etc.labeled, for example, with enzymes) for measuring the complexes in the step C (hereinafter, referred to as washing of the first solid phase) and by washing the second solid phase before conducting the step C after trapping the complexes eluted from the first solid phase in the step B, that is, the second solid phase contacted with labeled specifically binding substances slightly remaining in the eluate of the complexes from the first solid phase (hereinafter, referred to as washing of the second solid phase). The present invention has a feature that the number of times for washing actually used is determined on the bases of 1) the number of times for washing required theoretically calculated by taking into consideration a washing effect of the eluent of the complexes on the first solid phase, 2)the number of times for washing minimized by combining the numbers of times for washing the first and second solid phases, and 3) those numbers corrected on safety rate(Table 2).

When the volumes of the reaction solution containing a labeled specifically binding substance (hereinafter, referred to as A) incubated with the first solid phase and the eluent/the eluate of the complexes incubated with the first/ the second solid phases are both 100 µL and the volume of washing solution is 400 µL, the concentrations or the amounts of A in various steps are as follows. The concentration of A in the first washing solution of the first solid phase is about 1/(1.5 x 10²) of the concentration of A in the reaction solution incubated with the first solid phase. The concentration of A in the eluate after one washing of the first solid phase is about 1/(6 x 10³) of the concentration of A in the reaction solution. The concentration of A in the first washing solution of the second solid phase incubated with the eluate after one washing of the first solid phase is about 1/(9 × 10⁵) of the concentration of A in the reaction solution and the amount of A remaining on the second solid phase after removal of this first washing solution is about 1/(3.6 × 10⁷) of the amount of A in the reaction solution, indicating that, for example, even using as high a concentration as 10 pmol/mL of A, the amount of remaining A is about 2.8 × 10⁻²⁰ mol. Therefore, a highly sensitive assay is possible by one washing each of the first and second solid phases.

The concentration of A in the second washing solution of the first solid phase is about 1/(2.25 × 10⁴) of the concentration of A in the reaction solution. The concentration of A in the eluate after two washings of the first solid phase is about 1/(9 × 10⁵) of the concentration of A in the reaction solution. The amount of A remaining on the second solid phase after removal of the eluate following two washings of the first solid phase is about 1/(3.6 × 10⁷) of the amount of A in the reaction solution, which is equal to that after one washing each of the first and second solid phases described above. Therefore, two washings of the first solid phase provide a highly sensitive assay even without washing of the second solid phase.

Further, with two washings of the first solid phase and one washing of the second solid phase, the concentration of A in the first washing solution of the second solid phase is about 1/(1.35 × 10⁸) of the concentration of A in the reaction solution and the amount of A remaining on the second solid phase after removal of the first washing solution is about 1/(5.4 × 10⁹) of the amount of A in the reaction solution, ensuring a highly sensitive assay with high reproducibility and precision.

For example, even using as high a concentration as 100 pmol/mL of A, the amount of A remaining after two washings of the first solid phase and one washing of the second solid phase is only about 1.9 × 10⁻²¹ mol and, therefore, a highly sensitive assay is possible at zeptomolar levels.

Risk of low reproducibility and precision in the range close to the detection limit can be further lowered by using larger volumes of eluent or/and washing solution relative to the surface area of the solid phase.

Even when a solid phase of small or fine particles such as magnetic beads is used in the assay of the present invention, highly sensitive assay is still possible with the number of times for washing described above. However, when a solid phase of small or fine particles with large surface area (Japanese Patent Application No. 2001-140417) or/and a specifically binding substance of high concentration (Japanese Patent Application No. 2001-170186) which provides an ultra-rapid and ultra-sensitive assay is/are used, a highly sensitive assay with high reproducibility and precision is possible with two or three washings of the first solid phase and one or two washings of the second solid phase. In these conventional washings, solid phases of small or fine particles dispersed in washing solution are washed (hereinafter, referred to as dispersed washing) but, the time for washing can be shortened for more rapid and highly sensitive assays by washing solid phases of small or fine particles accumulated without dispersion (hereinafter, referred to as accumulated washing).

As hereunder, illustrated is a common solid phase assay method making it possible to lessen the cost of the non-competitive complex-transfer assay method and reduce the size of apparatuses for the assay.

For measuring an analyte by the non-competitive complex-transfer assay method, at least two kinds of solid phases, i.e. a first and a second solid phases, are required. Each of these first and second solid phases used may be different depending on the type of analytes. In the present invention, however, only two kinds of solid phases - a first and a second solid phases - which are common for different analytes are used.

For example, any of solid phases on which antihapten antibodies, haptens, DNAs, ion-exchange substances, etc. have been insolubilized may be used as the first solid phase, and the complexes of analytes with their specifically binding substances can be formed through at least one bond of antihapten antibody-hapten bonds, DNA hybrid bonds, ionic bonds, etc. Various compounds such as 2,4-dinitrophenyl group and biotin may be used as hapten. The present invention is not limited by these examples.

Solid phases on which avidin, streptavidin, antihapten antibodies different from those on the first solid phase, DNAs different from those on the first solid phase, etc. have been insolublized may be used as the second solid phase.

As hereunder, a highly sensitive luminescent assay method in which the luminescence intensity of the reagent solution for luminescent assay is measured after separation from the solid phase incubated with the reagent solution (Japanese Patent Application No. 2001-189419) is illustrated in detail.

In order to carry out the non-competitive complex-transfer assay method more rapidly and with higher sensitivity, label substances for assay in the complexes comprising analytes and their specifically binding substances (including modified or labeled ones) fixed on the second solid phase have to be measured more rapidly with higher sensitivity. The method used in the present invention is a method which is generally applied to the case where label substances for assay fixed on solid phases are measured by measuring the amount of luminescent substances produced in their presence more rapidly with higher sensitivity. More particularly, it is a rapid luminescent assay method, measuring the luminescence intensity of the reagent solution for luminescent assay after separation from the solid phases which substances producing luminescent substances on contact with the reagent solution or regulating their production have been fixed on and incubated with the reagent solution. Consequently, it is possible to measure label substances for assay on the solid phases more rapidly with higher sensitivity without limitation by type, shape, size, quantity and quality of solid phase, fixing method ,etc. For example, when the luminescence intensity of the reagent solution for luminescent assay (for example, a solution containing a dioxetane derivative as a luminescent substrate) is measured after contact with alkaline phosphatase as a label substance for assay bound to magnetic beads with a large surface area, namely, in a large quantity in assays such as immunoassays, DNA assays, etc. no highly sensitive assay is possible due to quenching by magnetic beads. However, quenching is eliminatated making a highly sensitive assay possible, when the luminescence intensity is measured after separation of the reagent solution for luminescent assay from the magnetic beads following the contact. Accordingly, the means of the present invention can be applied for carrying out the non-competitive complex-transfer assay method more rapidly with higher sensitivity.

In the method of the present invention, the luminescence intensity of the reagent solution for luminescent assay is measured after its separation from the solid phases which label substances for assay have been fixed on and contacted with the reagent solution. Although it is a feature of the present invention not to use a method for releasing label substances for assay from the solid phase, label substances for assay may be dissociated from the solid phase by the reagent solution for luminescent assay.

The reagent solution for luminescent assay according to the present invention is a solution in which a luminescent substance is produced on contact with label substances for assay fixed on the solid phase or its production is regulated, making it possible to measure label substances for assay by measuring the amount of the luminescent substance produced. For enzymes as label substance for assay, the reagent solution for luminescent assay is a solution containing a luminescent substrate which is converted to a luminescent substance by enzymatic action, i.e. a luminescent substrate solution. For example, for alkaline phosphatase (ALP) as label substance for assay, a luminescent substrate solution containing a dioxetane derivative (such as 2-chloro-5-(4-methoxyspiro{1,2-dioxetane -3,2'-(5'-chloro)- tricyclo[3,3,1,1,3,7]decan} - 4-yl)-1- phenylphosphate) as a luminescent substrate is the reagent solution for luminescent assay. A practically applicable solution of this compound is commercially available from Tropix as CDP-Star solution. Practically applicable solutions of similar compounds are also commercially available as the reagent solution for luminescent assay of ALP and β-D-galactosidase from Tropix (I. Bronstein, et al., *Journal of Bioluminescence and Chemiluminescence,* Vol. 4, 99-111 (1989)). Luminescent substances are produced from these compounds by hydrolytic activities of ALP, β-D-galactosidase, etc. and, therefore, it is possible to determine the amount of label substances for assay by measuring the amount of the luminescent substances produced. In cases where label substances for assay are substances which can regulate the activity of these enzymes, the amount of label substances can be determined by measuring the amount of luminescent substances produced under regulation after addition of a solution of the above compounds or similar compounds as a reagent solution for luminescent assay to a mixture of label substances and enzymes.

Measurement of luminescent intensity in the present invention can be carried out using various kinds of commercially available apparatuses. Examples of the apparatus are MLX^{TM} Microplate Luminometer (Dynex Technologies, Inc., Chantilly, VA) and Lumicounter 2500 (Microtec Co., Ltd., Chiba, Japan). There is no limitation by stage, time, frequency, method, etc. for measurement of luminescence intensity.

Although there is no limitation for the volume of the reagent solution for luminescent assay used, performance of assay is easy with sufficiently large volumes for covering all or nearly all of the solid phase surface on which label substances for assay have been fixed. Although there is no limitation for the temperature in contacting the reagent solution for luminescent assay with the solid phase, optimum temperatures for luminescent assay are preferred.

It is desirable that the reagent solution for luminescent assay is uniformly contacted with the solid phase surface by shaking, stirring, etc. but there is no limitation for the method of contacting.

Although there is no limitation for the time from contacting the reagent solution for luminescent assay with the solid phase until their separation, an appropriate time may be chosen by examination of the time course of changes in luminescence intensity after the contacting in advance of assay.

There is no limitation for the method of separating the reagent solution for luminescent assay from the solid phase, and various methods may be used depending upon type, shape, size, etc. of the solid phase. For separation from the solid phases in shapes of tubes, balls, etc., the reagent solution for luminescent assay is simply sucked with pipettes or the like. From magnetic beads, it can be separated by magnet, centrifugation, filtration, etc. From latexes, it can be separated by centrifugation, filtration, etc. For an efficient separation of the luminescent substance from the solid phase, an appropriate solvent or solution may be added follow by stirring etc. and subjected to a separating operation.

When the label substance for assay is a substance which regulates the production of luminescent substance, the reagent solution for luminescent assay has to be separated from the solid phase after stopping the production of luminescent substance. For example, when the label substance for assay is an inhibitor of alkaline phosphatase, the reagent solution for luminescent assay is separated from the solid phase after stopping the production of luminescent substance by addition of EDTA or the like.

Luminescence intensity is measured after transfer of the reagent solution for luminescent assay separated from the solid phase to tubes, cells or the like which are suitable for apparatuses used. The reagent solution for luminescent assay may be separated from fine particle solid phases such as magnetic beads and latexes in tubes, cells, etc. suitable for apparatuses used, simplifying the measuring operation. Since luminescence intensity of the reagent solution for luminescent assay separated from the solid phase on which the labeled substance for assay has been fixed decreases with the lapse of time, the operation from the separation until measurement of luminescence intensity is carried out preferably as rapidly as possible. Previous examination of the time course of decrease in luminescence intensity of the reagent solution for luminescent assay separated from the solid phase is helpful for performance. There is no limitation for the volume used for measurement of the luminescence intensity of the reagent solution for luminescent assay separated from the solid phase and a part or all of it may be used.

With smaller volumes of the reagent solution for luminescent assay to be contacted with the solid phase, nonspecific luminescence intensity is lower, leading to more sensitive assays. However, for fully ensuring the increase in luminescence intensity after the contact, sufficient amounts of luminescent substrate etc. have to be supplied to all surfaces of the solid phase on which label substances for assay have been fixed. For example, polystyrene balls are randomly rotated in a test tube containing small volumes of the reagent solution for luminescent assay so as to continuously supply the luminescent substrate, etc. onto all surfaces of the polystyrene balls (E. Ishikawa, Ultrasensitive and Rapid Enzyme Immunoassay, p. 141-176, Elsevier, Amsterdam, 1999 mentioned hereinabove already).

Furthermore, after contacting the solid phase with sufficient volumes of the reagent solution for luminescent assay, it is possible to remove most of the reagent solution leaving only minimum volumes required for measurement of slight amounts of label substance for assay and to collect the luminescent substance produced in small volumes of solvent or solution with low non-specific luminescence (cf. Japanese Patent Application No. 2001-062612; Title of the Invention: Method for Measuring the Substance Fixed on the Solid Phase). After this, luminescence intensity is measured according to the technique of the present invention. Namely, luminescence intensity can be measured after separation, from the solid phase, of the solvent or solution showing little non-specific luminescence in which the luminescent substance has been collected, further improving the sensitivity.

As hereunder, all the steps for the non-competitive complex-transfer assay method are more specifically illustrated, although not limited by the following specific examples.

### (Case Where the Analyte is Antigen)

The complex (es) of antigen as an analyte with antibody (ies) as its specifically binding substance(s) is formed on the first solid phase, for example, through a bond of hapten-antihapten antibody. Firstly, an antihapten antibody is insolubilized on a solid phase (first solid phase) followed by binding a haptenized antibody.

The antigen can be trapped on the first solid phase with a larger surface area more rapidly and more completely. Washing of the first solid phase at this stage removes interfering substances giving better results in many cases. However, washing at this stage is not an object of the present invention.

Further, by allowing a higher concentration of enzyme-labeled antibody to react with the antigen, the complex comprising the three components including haptenized antibody (2), the antigen (3) and enzyme-labeled antibody (4) is formed in a larger amount and more rapidly on the first solid phase(1) on which antihapten antibody has been insolubilized.

The amount of (3) trapped on the first solid phase and the amount of the complex comprising (2), (3) and (4) formed can be measured from the amount of (4) bound to the solid phase. The amount of (3) trapped can also be measured from decrease in the amount of (3) in the reaction solution.

Reaction order of (1), (2), (3) and (4) can be varied. In the first variation, a reaction product of (1) and (2) and that of (3) and (4) separately formed by previous incubations are allowed to react to form the complex comprising (2), (3) and (4) on (1).

By allowing (3) to react with higher concentrations of (4), the complex of (3) with (4) can be formed more efficiently within a shorter time and more completely and, with larger surface areas of (1), the complex of (3) with (4) can be trapped on (1) more rapidly and more completely.

In the second variation, (2) and (3) are allowed to react, then with (1) and finally with (4) to form the complex comprising (2), (3) and (4) on (1).

The complex of (2) with (3) is formed more rapidly and more completely using higher concentrations of (2), then trapped on (1) of larger surface areas more rapidly and more completely and finally allowed to react with (4) of higher concentrations providing an ultra-rapid and ultra-sensitive assay of the antigen.

The amount of the complex of (2) and (3) formed can be measured from the enzymatic activity bound to (1) after the reaction with (4).

In the 3rd, 4th or stir variation, the reaction is allowed in the order of (2), (3) and (4) or in the order of (3), (4) and (2) or the three are allowed to react simultaneously to form the complex comprising the three and then the complex formed is trapped on (1). With higher concentrations of (2) and (4), the complex comprising the three is formed more rapidly, more efficiently and more completely and is trapped on (1) of larger surface areas, providing an ultra-rapid and ultra-sensitive assay of the antigen.

Before moving from the step A to the step B, (1) contacted with (4) is washed once or twice. With larger volumes of washing solution, washing is more effective. With (1) of small or fine particles having large surface areas or/and (4) of high concentrations, assay results with high reproducibility and precision are obtained with higher probability by washing (1) dispersed in the washing solution twice or thrice. Washing (1) accumulated once and (1) dispersed once or twice further shorten the time required for washing, providing an ultra-rapid and ultra-sensitive assay.

### (Case Where the Analyte is Antibody)

The complex of antibody as an analyte with the corresponding antigen is formed on the first solid phase, for example, through a bond of hapten and antihapten antibody. The first solid phase (5) on which antihapten antibody has been insolubilized, haptenized antigen (6), antibody (7) and enzyme-labeled antigen (8) are successively allowed to react entirely in the same way as in the successive reaction of (1), (2), (3) and (4) for the antigen assay except that antigen and antibody are mutually replaced. A similar successive reaction is possible with enzyme-labeled anti-immunoglobulin antibody (9) substituted for (8). Furthermore, (6), (7) and (8) are simultaneously allowed to react and form the complex comprising the three on (5) entirely in the same way as the formation of the complex comprising (2), (3) and (4) on (1) by simultaneous reaction except that antigen and antibody are mutually replaced.

### (Case Where the Analyte is RNA and DNA)

RNA as an analyte can also be measured by converting to DNA.

The complex comprising DNA as an analyte, DNA for trapping and DNA for assay is formed on the first solid phase through a bond of hapten-antihapteri antibody. It is possible to form the complex comprising haptenized DNA for trapping, DNA as an analyte and biotinylated (or enzyme-labeled) DNA for assay on the first solid phase as in the antigen assay. Formation of the complex with DNA as an analyte is analogous to that with antigen as an analyte by binding of haptenized antibody for trapping and enzyme-labeled antibody for assay to two different sites of antigen molecule.

However, it is different from that with antibody as an analyte. Namely, haptenized antigen for trapping and enzyme-labeled antigen for assay bind to two different sites of antibody molecule, but epitopes binding to the two different sites are identical. Formation of the complex with DNA as an analyte having more than 2 sites of identical base sequence is, of course, similar to that with antibody as an analyte.

### (Formation of Complexes using Bonds Other Than Antihapten Antibody-Hapten Bond)

The complex of an analyte with its specifically binding substance(s) (including modified or labeled ones) can be formed on the solid phase (the first solid phase) through disulfide bond (-S-S-), ionic bond, DNA hybrid or physical adsorption in place of hapten-antihapten antibody bond. For use of disulfide bond and physical adsorption, specifically binding substances for trapping analytes are preferably fixed on the first solid phase through these bonds in advance of other reactions.

Preferably in advance of other reactions prepared are, for example, (first solid phase)-(bovine serum albumin)-S-S-(antibody) and (first solid phase)-(physically adsorbed antibody) for the antigen assay, (first solid phase)-(bovine serum albumin)-S-S-(antigen) and (first solid phase)-(physically adsorbed antigen) for the antibody assay and (first solid phase)-(bovine serum albumin)-S-S-(DNA) for the DNA assay.

The step B of the assay method in the present invention is performed by the methods corresponding to those by which the complexes of analytes and their specifically binding substances (including modified and labeled ones) are formed and fixed on the first solid phase in the step A (E. Ishikawa, Ultrasensitive and Rapid Enzyme Immunoassay, Laboratory Techniques in Biochemistry and Molecular Biology, Vol. 27, S. Pillai, P. C. van der Vliet eds., pp. 71-191, 1999; cited already).

The complexes which are formed on the first solid phase in the step A through hapten-antihapten antibody bonds, disulfide bonds, ionic bonds, DNA hybrids and physical adsorption are eluted using high concentrations of haptens or their derivatives, reducing agents such as 2-mercaptoethylamine, high concentrations of salts, high temperatures and detergents, respectively. The complexes formed on the first solid phase through two or more bonds are eluted by two or more methods. For example, the complexes formed through antihapten antibody-hapten bonds on the first solid phase on which antihapten antibodies have been insolubilized by physical adsorption can be eluted using both high concentrations of haptens or their derivatives and detergents. (S. Ishikawa, et al., *Anal. Lett.,* Vol. 33, No. 11, pp. 2183-2196, 2000).

The use of monoclonal antihapten antibodies is suitable for formation and fixation of the complexes comprising analytes and their specifically binding substances (including modified or labeled ones), on the first solid phase through hapten-antihapten antibody bonds (Japanese Patent Application No. 2000-400853). Monoclonal antihapten antibodies can be chosen so that the complexes are eluted from the first solid phase more completely and more rapidly. Among them, monoclonal anti-2,4-dinitrophenyl group antibody is suitable.

The complexes comprising haptenized antigens, antibodies and enzyme-labeled antigens can be fixed (trapped) on the second solid phases on which anti-immunoglobulin antibodies have been insolubilized.It is desirable that affinity-purified polyclonal or highly purified monoclonal anti-immunoglobulin antibodies with high affinity are insolubilized on the second solid phase in as much amounts as possible, and that temperature, pH, shaking method, type of ion and ionic strength, etc. are optimum. The complexes are trapped on the second solid phase more completely within shorter periods of time using, in addtion to the above conditions, larger surface areas of the second solid phase as compared with the volume of the reaction solutions containing the complexes, namely, the volume of the eluates, providing ultra-rapid and ultrasensitive antibody assays (Japanese Patent Application No. 2001-140417). The complexes containing specifically binding substances biotinylated such as (haptenized biotinylated antibody)-(antigen)-(enzyme-labeled antibody), (haptenized biotinylated antigen) - (antibody)-(enzyme-labeled antigen), (HS- biotinylated antibody) - (antigen) - (enzyme-labeled antibody) and (haptenized biotinylated antigen) - (antibody) - (enzyme- labeled anti-immunoglobulin antibody) can be trapped on the second solid phase on which avidin or streptavidin has been insolubilized. Desirably, various conditions described above are optimum for trapping. Using larger surface areas of the second solid phase in addition to optimal conditions described above, the complexes are trapped on the second solid phase more completely within shorter periods of time. For trapping the complexes eluted using detergents, anti- immunoglobulin antibody, avidin, streptavidin, etc. have to be insolubilized on the second solid phase through covalent bonds.

Illustrated above is trapping on the second solid phase of the complexes which have been released into the eluates. In contrast to this, elution and trapping on the second solid phase can be performed simultaneously. For example, the complexes formed on the first solid phase through hapten-antihapten antibody bonds are simultaneously eluted and trapped by simultaneous incubation of both the first and the second solid phases in the eluent containing haptens of high concentrations. In this case, the trapping rate depends on the elution rate as well. Therefore, the trapping rate is tested using the following complexes or substances: the same complexes as those eluted and transferred from the first solid phase to the second solid phase in the step B, part of those complexes or substances contained in those complexes and used for transfer and fixation. The second solid phase used in the present invention has such large surface areas that the amounts of the above complexes, their part or the above substances trapped on the second solid phase by incubation for as short a time as 3 minutes, 2 minutes or 1 minutes as in the step A are 60% or more, preferably 70% or more, more preferably 80% or more and most preferably 90% or more of the maximum amount (defined as 100%) trapped by incubation for as sufficiently long a time as 10 minutes, 30 minutes or 60 minutes. For example, for trapping and fixation of the complexes such as (haptenized antigen)-(antibody)-(enzyme-labeled antigen) on the second solid phase on which anti-immunoglobulin antibody has been insolubilized, the second solid phase used has such a large surface area that the complexes or immunoglobulins are trapped efficiently within a short time as described above. Time from the start of simultaneous contact of the first and second solid phases with the eluent until the start of contact of the second solid phase with washing solution after separation from the eluate shall be 10 seconds to 3 minutes.

The second solid phase before moving to the step C from the step B is washed once after one washing of the first solid phase and, after two washings of the first solid phase, there is no need to wash the second solid phase, although assay results with high reproducibility and precision are obtained by one washing of the second solid phase. When small or fine particles with large surface areas are used as the second solid phase or/and when high concentrations of labeled specifically binding substances are used, one or two washings of the second solid phase give assay results with high reproducibility and precision. The volume of washing solution is preferably larger. Although washing of small or fine particle solid phases dispersed in washing solution is highly effective, washing of those accumulated without dispersion shortens the time required for washing in compensation for less washing efficiency, providing more rapid and sensitive assay.

The step C for the non-competitive complex-transfer assay method used in the present invention can be carried out by known methods corresponding to the states of the complexes trapped on the second solid phase in the step B(E. Ishikawa, Ultrasensitive and Rapid Enzyme Immunoassay, Laboratory Techniques in Biochemistry and Molecular Biology, Vol. 27, S. Pillai, P. C. van der Vliet eds., Elsevier, Amsterdam, pp. 79-120, 1999; cited already). However, the present invention is not limited to the known methods. The complexes on the second solid phase such as (second solid phase)-(antihapten antibody)-(haptenized antigen)-(antibody), into which labels such as enzymes have not been introduced, are measured after binding an enzyme-labeled anti-immunoglobulin antibody. However, in many cases, labels for assay are introduced in the step A. Analytes can be measured by measuring these labels for assay on the second solid phase by the corresponding methods without dissociation. However, the assay of analytes is possible even by measuring labels alone, part of the complexes or whole complexes dissociated from the second solid phase,as far as sensitive measurements are not interfered with. The activity of enzymes is measured by colorimetry,y, fluorometry, luminescent assay, ESR intensity measuring method, etc. Among enzymes as label for assay, alkaline phosphatase is suitable, and dioxetane derivatives are suitable as its luminescent substrate. Commercially available CDP-star ready to use with Sapphire II (Tropix Inc., Bedford, MA) is suitable as a luminescent substrate for alkaline phosphatase. Fluorescent and luminescent substances can be measured by the known methods measuring fluorescence and luminescence intensities, respectively. However, the present invention is not limited to the known methods.

In luminescent assay of label substances for assay, for example, enzymes on the second solid phase of small or fine particles, high sensitivity is obtained by measuring the luminescence intensity of the reagent solution for luminescent assay after separation from the second solid phase following incubation of the both (Japanese Patent Application No.2001-189419).

As hereunder, a highly sensitive solid phase assay method for analytes contained in whole blood or mixed with whole blood is illustrated.

Use of whole blood as a sample saves time for separation of serum and plasma and, therefore, greatly shortens the time from collection of blood until utilization of assay results as compared with use of serum and plasma as a sample. This not only makes therapies such as emergency operations easy but also removes the burden of patients such as second comings and reexaminations at later dates due to a long time required for blood tests. However, whole blood interferes with the conventional known solid phase assay methods, excluding its use as a sample.

The present inventors have found that analytes can be measured without interference by whole blood with non-competitive complex-transfer assay method in which the complex(es) of an analyte with its specifically binding substance(s) (including modified or labeled ones) is formed on a solid phase (a first solid phase), the complex(es) on the first solid phase is transferred to another solid phase (a second solid phase) and the complex(es) on the second solid phase is measured for measuring the analyte, and have accomplished the present invention. Namely, identical assay results are obtained by the non-competitive complex-transfer assay method described above using any of whole blood, serum and plasma as a sample.

Whole blood in the present invention indicates blood collected *per se.* There is no limitation for the method of collecting blood. Since blood after collection coagulates in containers outside living bodies, anticoagulants are added in many cases. However, there is no limitation for the state of blood after collection such as addition or non-addition of anticoagulants, time, temperature and methods of storage, type of additives,etc. Whole blood dried on filter paper is also within a scope of the present invention.

The analytes in the present invention include all substances contained in or mixed with whole blood for which specifically binding substances are available. Specifically binding substances and substances which specifically binding substances are available for are illustrated above.

The non-competitive complex-transfer assay method using whole blood as a sample according to the present invention can be carried out by various known methods (E. Ishikawa, Ultrasensitive and Rapid Enzyme Immunoassay, Laboratory Techniques in Biochemistry and Molecular Biology, Vol. 27, S. Pillai, P. C. van der Vliet, eds., pp. 79-191, Elsevier, Amsterdam, 1999) except that whole blood is substituted for the conventional serum and plasma samples. However, the present invention is not limited to the known methods.

The present invention further provides an ultra-rapid and ultra-sensitive assay method for analytes contained in or mixed with whole blood by at least one of the following methods: a method in which, using solid phases with larger surface areas, analytes or the complexes are trapped on the first solid phase or/and the second solid phase more completely and more rapidly in the step A-1 or/and the step B-2 as described above (Japanese Patent Application No. 2001-140417), a method in which, using higher concentrations of specifically binding substances (including modified or labeled ones), the complexes are formed more completely and more rapidly in the step A-2 (Japanese Patent Application No. 2001-170186), a method in which, using monoclonal antihapten antibody with higher ability, the complexes are eluted from the first solid phase more completely and more rapidly in the step B-1 (Japanese Patent Application No. 2000-400853), a method in which, using minimum numbers of times for washing of solid phases, the steps A-3 and B-3 are carried out more rapidly (Japanese Patent Application No. 2001-204964) and a highly sensitive method for measurement of luminescence intensity of a reagent solution for luminescent assay after separation from the solid phase (Japanese Patent Application No. 2001-289419).

The present invention covers solid phases, reagents, etc. for carrying out assay methods illustrated above and further covers assay kits containing at least one of reagents or/and solid phases used in the present invention being exemplified by the said solid phases and buffers, blocking solutions, washing solutions, substrate solutions, antibodies, haptens, etc. The present invention still further covers assay. Systems containing solid phases, reagents and automated softwares, for carrying out the assay method illustrated above.

Although the above is illustrated by way of exemplifications, the present invention is not limited by these exemplifications.

### Examples

The present invention will be specifically illustrated by the following examples, although the present invention is not limited by those examples.

### Example 1

This example shows that the amount of the immune complex trapped on the magnetic beads increases with increased in their surface area.

### (Materials and Methods)

. Blocking solution
   10 mM sodium phosphate buffer,pH 7.0, containing 0.15M NaCl, 2.5 mM EDTA, 2.5 g/L bovine serum albumin, 10 g/L sucrose and 1 g/L NaN₃
. Washing solution for alkaline phosphatase (hereinafter, referred to as ALP)
   20 mM Tris.HCI buffer, pH 7.4, containing 0.15 M NaCl 0.1% Tween 20 and 0.1% NaN₃
. Triethanolamine (TEA) buffer
   0.1 M Triethanolamine-HCl buffer, pH 7.6, containing 0.1 mM ZnCl₂, 1 mM MgCl₂, 1 g/L bovine serum albumin and 0.05% NaN₃
. ALP obtained from
   Oriental Yeast Co., Ltd., Tokyo, Japan
. Monoclonal anti-HBsAg (human hepatitis B virus surface antigen) antibodies 649 and 85 and monoclonal anti-2,4-dinitrophenyl group (hereinafter, referred to as DNP) antibody-1753, International Reagents Corporation, Kobe, Japan
. Magnetic beads
   MG 205, diameter: 880 nm, specific gravity: 1.3, JSR Corporation, Tokyo, Japan. The surface area of 1 mg of the magnetic beads calculated on the assumption that they are spheres is 52.45 cm².
. Fluorescent substrate solution for ALP
   Attophos™ Test Kit, JBL Scientific Inc., San Luis Obispo, CA
. Microplate
   Fluoro Nunc™ Module Plate, F16 Maxisorp Black, NuncA/S, DK-4000 Roskilde, Denmark
. DNP-biotin-anti-HBsAg Fab' and ALP-anti-HBsAg Fab'
   2,4-dinitrophenylated-biotinylated bovine serum albumin-anti-HBsAg Fab'-649 (DNP-biotin-anti-HBsAg Fab') and ALP-anti-HBsAg Fab'-85 (ALP-anti-HBsAg Fab') were prepared by a known method using the reaction of maleimide groups with thiol groups (E. Ishikawa, Ultrasensitive and Rapid Enzyme Immunoassay, Laboratory Techniques in Biochemistry and Molecular Biology, Vol. 27, S. Pillai, P. C. van der Vliet, eds., pp. 177-302, 1999).
. Magnetic beads on which anti-DNP IgG had been insolubilized
   Monoclonal anti-DNP IgG-1753 (Japanese Patent Application No. 2000-400853, filed on December 28, 2000 by International Reagents Corporation) was insolubilized on magnetic beads according to the directions of JSR Corporation, washed with blocking solution and stored in the same solution at 4°C.
. Formation of the Immune Complex
   DNP-biotin-anti-HBsAg Fab' (1 pmol), 0.48 pmol of ALP-anti-HBsAg Fab' and 0.05 IU of HBsAg were incubated in 120 µL of TEA buffer at 37°C for 10 minutes.
. Binding of the Immune Complex to the Magnetic Beads on which anti-DNP IgG had been insolubilized
   The solution described above (120 µL) containing the immune complex and 0.1, 0.2, 0.5 or 1 mg of the magnetic beads which anti-DNP IgG had been insolubilized on and washed with TEA buffer were incubated at room temperature for 1 minute, 2 minutes or 10 minutes (including 20 seconds for magnetic separation) and the magnetic beads were washed with 0.4 mL of washing solution for ALP three times by means of magnetic separation.
. Fluorophotometer
   CytoFluor® 4000 Multiwell Fluorescent Plate Reader, PerSeptive Biosystems, Inc., Framingham, MA
. Fluorometry of ALP Activity
   To the washed magnetic beads described above on which anti-DNP IgG had been insolubilized were added 200 µL of fluorescent substrate solution for ALP, followed by incubation at 37°C for 30 minutes. The fluorescence intensity of the whole supernatant separated from the magnetic beads by magnetic force was measured using wavelengths of 450 nm for excitation and 580 nm for emission analysis.

### (Results)

The results of Example 1 are shown in Table 3.

When 120 µL of reaction solution containing the immune complex comprising the three components, i.e. DNP-biotin-anti-HBsAg Fab', HBsAg and ALP-anti-HBsAg Fab' and the magnetic beads on which anti-DNP IgG had been insolubilized for trapping the immune complex were incubated for as short a time as 1 minute or 2 minutes, the amount of the immune complex trapped on the magnetic beads was shown to approach, by increasing the amount of the magnetic beads used, that is, the surface area of the magnetic beads used, the amount of the immune complex trapped when incubated for as long a time as 10 minutes. Namely, with 0.1 mg of the magnetic beads having a surface area of 5.2 cm², the amounts of the immune complex trapped by incubation for 1 minute and 2 minutes were 47.6% and 69.2% ,respectively, of the amount of the immune complex trapped by incubation for as long a time as 10 minutes (defined as 100%),but they were 71.8% and 91.3%, respectively, with 0.2 mg/10.4cm²., 97.3∼98.2% with 0.5mg/26 cm², and 99.4∼99.8% with 1 mg/52 cm².

### Example 2

This example shows the effect that the amount of the immune complex eluted from the magnetic beads on which anti-DNP IgG has been insolubilized and trapped on the magnetic beads on which streptavidin has been insolubilized increases with increase in the surface area of the last magnetic beads.

### (Materials and Methods)

Materials and methods used are the same as those described in Example 1 except those described below.
. Phosphate buffer
   0.1 M Sodium phosphate buffer, pH 7.5,containing 0.05% NaN₃
. Streptavidin
   Type II, Wako Pure Chemical Industries, Ltd., Osaka, Japan
. DNP-Lys solution
   TEA buffer containing 3 mM εN-2,4-dinitrophenyl-L-lysine
. Magnetic beads on which streptavidin had been insolubilized
   Biotinylated bovine serum albumin was prepared by a known method (E. Ishikawa, Ultrasensitive and Rapid Enzyme Immunoassay, Laboratory Techniques in Biochemistry and Molecular Biology, Vol. 27, S. Pillai, P. C. van der Vliet, eds., pp. 177-302, 1999), insolubilized on magnetic beads according to the directions of JSR Corporation and allowed to react with 30 µg/mL of streptavidin in phosphate buffer. The magnetic beads were washed with phosphate buffer and stored in blocking solution at 4°C.
. Binding of the immune complex to the magnetic beads on which anti-DNP IgG had been insolubilized
   The immune complex was trapped on the magnetic beads (0.5 mg) on which anti-DNP IgG had been insolubilized by 1minute incubation.
. Elution of the immune complex from the magnetic beads on which anti-DNP IgG had been insolubilized
   The magnetic beads described above on which anti-DNP IgG had been insolubilized followed by binding of the immune complex were incubated with 100 µL of DNP-Lys solution at room temperature for 1 minute and subjected to magnetic separation and the supernatant was used as an eluate.
. Binding of the immune complex to the magnetic beads on which streptavidin had been insolubilized
   The above eluate was incubated at room temperature for 1 minute, 2 minutes or 10 minutes (including 20 seconds for magnetic separation) with 0.1, 0.2 or 0.5 mg of the magnetic beads on which streptavidin had been insolubilized, and the magnetic beads were washed with 400 µL of washing solution for ALP three times by means of magnetic separation.
. Fluorometry of ALP activity
   The ALP activity bound to the above magnetic beads which streptavidin had been insolubilized on followed by binding of the immune complex was measured in the same manner as in Example 1.

### (Results)

The results of Example 2 are shown in Table 4.

When the immune complex formed by the method of Example 1 on the magnetic beads on which anti-DNP IgG had been insolubilized was eluted with DNP-Lys solution and the immune complex in the eluate was incubated with the magnetic beads on which streptavidin had been insolubilized for as short a time as 1 minute or 2 minutes, the amount of the immune complex trapped on the last magnetic beads was shown to approach, by increasing the amount of the last magnetic beads used, that is, the surface area of the last magnetic beads used, the amount trapped when incubated for as long a time as 10 minutes. Namely, with 0.1 mg of the last magnetic beads having a surface area of 5.2 cm² , the amount of the immune complex trapped by incubation for 1 minute and 2 minutes were 48.7% and 69.5%, respectively, of the amount of the immune complex trapped by incubation for as long a time as 10 minutes (being defined as 100%),but they were 73.1% and 93.4%, respectively, with 0.2 mg / 10.4 cm² and 97.1% and 101.6%, respectively, with 0.5 mg/26 cm².

### Example 3

This example shows that the sensitivity can be improved by trapping the immune complex on the first solid phase (magnetic beads on which anti-DNP IgG has been insolubilized) within as short a time as about 1 minute,.eluting it from the first solid phase within as short a time as about 1 minute and trapping it on the second solid phase (magnetic beads on which streptavidin has been insolubilized) within as short a time as about 1 minute.

### (Materials and Methods)

Materials and methods used are the same as those described in Examples 1 and 2 except those described below.
. Formation of the immune complex
   The immune complex was formed by incubation for 6 minutes.
. Binding of the immune complex to the magnetic beads on which anti-DNP IgG had been insolubilized
   The immune complex was bound to 0.1 mg and 0.5 mg of the magnetic beads on which anti-DNP IgG had been insolubilized by incubation for 1 minute (including 20 seconds for magnetic separation).
. Binding of the immune complex to the magnetic beads on which streptavidin had been insolubilized
   The immune complex eluted was bound to 0.1 mg and 0.25 mg of the magnetic beads on which streptavidin had been insolubilized by incubation for 1 minute (including 20 seconds for magnetic separation).
. Luminescent substrate solution for ALP
   CDP-star ready to use with Sapphire II, Tropix, Inc., Bedford, MA
. Apparatus for measuring luminescence intensity
   Lumicounter 2500, Microtec Co., Ltd., Chiba, Japan
. Luminescent assay of ALP activity
   Luminescent substrate solution for ALP (200 µL) added to the above magnetic beads which streptavidin had been insolubilized on followed by binding of, the immune complex was incubated at room temperature for 5 minutes and subsequently luminescence intensity of the whole supernatant obtained by magnetic separation was measured to obtain luminescence quantity for 0.1 second.

### (Results)

The results of Example 3 are shown in Table 5.

The sensitivity was shown to be improved by increasing the surface areas of the first solid phase and the second solid phase in the present invention. Namely, by increasing the amount of the magnetic beads used from 0.1 mg to 0.25∼0.5 mg, the assay sensitivity of HBsAg was improved 3.3-fold.

### (Comparative Example 1)

In this Comparative Example, HBsAg was measured by a known sandwich method using microplate as a solid phase and the result was compared with that of Example 3 according to the present invention.

### (Materials and methods)

Materials and methods used are the same as those in Examples 1, 2 and 3 except those described below.
. Microplate on which monoclonal anti-HBsAg IgG had been insolubilized
   Monoclonal anti-HBsAg IgG-649 in 100 µL of phosphate buffer (10µg/ml) was added to each welland allowed to stand at 4°C overnight. The microplates were washed with washing solution for ALP and stored at 4°C after addtion of 300 µL of blocking solution.
. Diluent solution of HbsAg and ALP-anti-HBsAg Fab'
   50 mM triethanolamine-HCl buffer, pH 7.0, containing 1 mM MgCl2, 0.1 mM ZnCl₂ and 1 g/L bovine serum albumin
. HBsAg sandwich method
   100 µ L of 0.5 IU/mL HBsAg were added to the microplates on which monoclonal anti-HBsAg IgG had been insolubilized and stirred at room temperature for 30 minutes. After washing with 200 µL of washing solution for ALP three times, 100 µL of 0.6 pmol/mL ALP-anti-HBsAg Fab' were added to the microplates and stirred at room temperature for 30 minutes followed by washing three times.
. Apparatus for luminescence measurement
   MLX™ Microplate Luminometer, Dynex Technologies, Inc., Chantilly, VA
. Luminescent assay for ALP activity
   To the washed microplates described above were added 200 µL of luminescent substrate solution for ALP used in Example 3 and incubated at 37°C for 10 minutes. Subsequently, luminescence intensity was measured for 0.2 second and a mean value for 0.01 second was obtained.

### (Comparative Example 2)

In this comparative example, HBsAg was measured by a known sandwich method using ferrite particles, labeled antibody and substrate solution of Luminescent Enzyme Immunoassay Reagents Lumipulse II (Fuji Rebio Inc., Tokyo, Japan) and the result was compared with that of Example 3 by the present invention.

### (Materials and methods)

Materials and methods used are the same as those in Example 1 except those described below.

A: 150 µg/mL ferrite particles on which anti-HBsAg antibody (rabbit) had been insolubilized (250 µL) and 100 µL of 0.5IU/mL HBsAg dissolved in TEA buffer were mixed and incubated at 37°C for 10 minutes. The ferrite particles were separated with a magnet, washed with 400 µL of washing solution for ALP once and incubated with 250 µL of 0.25 µg/mL ALP-labeled anti-HBsAg antibody (mouse) at 37°C for 10 minutes followed by washing with 400 µL of washing solution for ALP three times. The ferrite particles and 200 µL of the substrate solution were incubated at 37°C for 10 minutes in microplates and, without separating the ferrite particles with a magnet, luminescence intensity was measured in the same manner as in Comparative Example 1.

B: HBsAg was measured by a sandwich method using the solid phase, reagents and automatic assay apparatus of Lumipulse II HBsAg (Fuji Rebio Inc., Tokyo, Japan).

### (Results)

The results of Example 3 and Comparative Examples 1 and 2 are collectively shown in Table 6.

The sensitivity by the present invention in which the complex of DNP-biotin-anti-HBsAg Fab', ALP-anti-HBsAg Fab' and HBsAg was trapped on the first and the second solid phases by only one minute incubation was 14-fold higher than that by the known sandwich method in Comparative Example 1 in which HBsAg as an analyte was trapped on the microplate by incubation for as long a time as 30 minutes and 12- to 24-fold higher than that by the known sandwich method in Comparative Example 2 in which HBsAg as an analyte was trapped on the microplate by incubation for as long a time as 10 minutes.

Namely, the large-surface solid phase assay method in accordance with the present invention was shown to provide a far more rapid and far more sensitive assay method than the known sandwich methods.

### (Comparative Example 3)

In this comparative example, the time course of trapping HBsAg on the solid phase by the known sandwich method in Comparative Example 2 was examined and the result is compared with that of Example 4 which showed it by the present invention.

### (Materials and methods)

Materials and methods used are the same as those in Comparative Example 2A except those described below.

Ferrite particles to which anti-HBsAg antibody (rabbit) had been bound were incubated with HBsAg (0.5 IU) for 2, 3, 10 or 20 minutes and allowed to react with ALP-labeled anti-HBsAg antibody. The washed ferrite particles and the substrate solution were incubated at room temperature for 5 minutes and, without separating the ferrite particles with magnet, luminescence intensity was measured in the same manner as in Example 3.

### Example 4

This example shows the time course of trapping HBsAg on the solid phase by the present invention for comparison with that by the known sandwich method shown in Comparative Example 3.

### (Materials and methods)

Materials and methods are the same as those in Example 1 except those described below.
. Binding of DNP-biotin-anti-HBsAg Fab' to magnetic beads (first solid phase) on which anti-DNP IgG had been insolubilized
   The magnetic beads (0.5 mg,first solid phase) on which anti-DNP IgG had been insolubilized were incubated with DNP-biotin-anti-HBsAg Fab' (5 pmol) dissolved in 50 µL of TEA buffer at room temperature for 1 minute and , after magnetic separation, washed with 400 µL of washing solution for ALP twice.
. Binding of HBsAg to the first solid phase
   The first solid phase to which DNP-biotin-anti-HBsAg Fab' had been bound were incubated with HBsAg (0.5 IU) dissolved in 100 µL of TEA buffer at room temperature for 1 minute, 2 minutes, 5 minutes or 10 minutes and washed with 400 µL of washing solution for ALP twice.
. Binding of ALP-anti-HBsAg Fab' to the first solid phase
   The first solid phase to which HBsAg had been bound was incubated with ALP-anti-HBsAg Fab' (0.48 pmol) dissolved in 100 µL of TEA buffer at room temperature for 1 minute and washed with 400µL of washing solution for ALP three times.
. Fluorometry of ALP activity
   Fluorometry was performed in the same manner as in Example 1 except that ALP activity bound to the first solid phase with which ALP-anti-HBsAg Fab' had been allowed to react was incubated with fluorescent substrate solution for ALP at 37°C for 10 minutes followed by addtion of 50µL of 0.1M EDTA as a stopping solution for ALP reaction and the whole supernatant magnetically-separated was transferred to a microplate.

### (Results)

The results of Comparative Example 3 and Example 4 are collectively shown in Table 7.

HBsAg was trapped on the solid phase far more rapidly by the method of the present invention in which 0.5 mg of magnetic beads in 100 µL and HBsAg were incubated (Example 4) than by a known sandwich method (Comparative Example 3) using 37.5 µg of ferrite particles in 350 µL. Namely, the amount trapped within 2 minutes in the known sandwich method was about 30% of the amount trapped within 10 to 20 minutes. In contrast, in the method of the present invention, 55.8% and 71.1% were trapped within 1 minute and 2 minutes, respectively, when the amount trapped within 10 minutes was defined as 100%.

Advantages of the present invention shown by Examples 1 to 4 and Comparative Examples 1 to 3

In a method where the complex of a substance to be assayed (analyte) and its specifically-binding substance(s) (including modified or labeled ones) is formed on a solid phase (the first solid phase), then the complex is transferred to another solid phase (the second solid phase) and finally the complex on the second solid phase is measure by which the analyte is measured, the incubation time of the analyte or the complex with the solid phase was shortened by using larger surface areas of the first solid phase or/and the second solid phase incubated with the reaction solution containing the analyte or the complex as compared with the volume of the reaction solution, providing a rapid and highly-sensitiveassay. Further, this was combined with shortening the time required for elution of the complex from the first solid phase using monoclonal antihapten antibody with high ability, or/and with measurement of luminescence intensity of the reagent solution for luminescence assay after separation from the second solid phase, providing a rapid and highly-sensitive assay.

### Example 5

In this example, the amount of the complex formed of an analyte, HBsAg and its specifically-binding substance, DNP-biotin-anti-HBsAg Fab' is shown to increase by raising the concentration of the specifically-binding substance, DNP-biotin-anti-HBsAg Fab'.

### (Materials and methods)

Materials and methods used are the same as those in Example 1 except those described below.
. Formation of the immune complex
   DNP-biotin-anti-HBsAg Fab' (0.5, 2.5 or 5 pmol) and 0.5 IU of HBsAg were incubated in 110 µL of TEA buffer at room temperature for 5 seconds, 1 minute or 10 minutes.
. Binding of the immune complex to the magnetic beads on which anti-DNP IgG had been insolubilized
   110 µL of the reaction solution containing the immune complex described above and 1 mg of the magnetic beads, on which anti-DNP IgG had been insolubilized followed by washing with TEA buffer were incubated at room temperature for 1 minute (including 20 seconds for separation and accumulation of the magnetic beads) and the magnetic beads were washed with 0.4 mL of washing solution for ALP twice.
. Binding ofALP-anti-HBsAg Fab' to the magnetic beads on which anti-DNP IgG had been insolubilized
   ALP-anti-HBsAg Fab' (4.8 pmol/mL, 100 µL) diluted with TEA buffer and the magnetic beads after washing were incubated at room temperature for 1.5 minutes (including 30 seconds for separation and accumulation of the magnetic beads) and the magnetic beads were washed with 0.4 mL of washing solution for ALP three times.
. Fluorometry of ALP activity
   ALP activity bound to the washed magnetic beads was measured in the same manner as in Example 4 except that 50 µL of 0.36 M K₂HPO₄ were used as a stopping solution for the ALP reaction.

### (Results)

The results of Example 5 are shown in Table 8.

The amount of the immune complex formed when DNP-biotin-anti-HBsAg Fab' and HBsAg were incubated for as short a time as 65 seconds and 2 minutes was shown to approach the amount of the immune complex formed when incubated for as long a time as 11 minutes by raising the concentration of the DNP-biotin-anti-HBsAg Fab'. Namely, with 0.5 pmol of DNP-biotin-anti-HBsAg Fab', the amounts of the immune complex formed by incubation for 65 seconds and 2 minutes were 40.5% and 62.0% , respectively, of the amount formed by incubation for 11 minutes but, they were 70.3% and 103.6%, respectively, with 2.5pmol and 71.4% and 95.6%, respectively, with 5pmol.

### Example 6

In this example, the amount of the immune complex of DNP-biotin-anti-HBsAg Fab', HBsAg and ALP-anti-HBsAg Fab' formed is shown to increase by raising the concentration ofALP-Anti-HBsAg Fab'.

The materials and methods used in Example 5 were used except that the incubation times were 1 minutes for 1 pmol of DNP-biotin-anti-HBsAg Fab' with 0.5 IU of HbsAg, 1.5 minutes (including 30 seconds for separation and accumulation of the magnetic beads) with 0.5 mg of the magnetic beads on which anti-DNP IgG had been insolubilized and 30, 60 or 600 seconds (including 20 seconds for separation and accumulation of the magnetic beads) with 0.48, 0.96 or 1.92 pmol of ALP-anti-HBsAg Fab':

### (Results)

The results of Example 6 are shown in Table 9.

By raising the concentration of ALP-anti-HBsAg Fab', the amount of the immune complex formed when the magnetic beads which anti-DNP IgG had been insolubilized on followed by binding of DNP-biotin-anti-HBsAg Fab' and HBsAg were incubated with ALP-anti-HBsAg Fab' for as short a time as 30 or 60 seconds was shown to approach the amount of the immune complex formed when incubated for as long a time as 600 seconds. Namely, with 0.48pmol of ALP-anti-HBsAg Fab', the amounts formed by incubation for as short a time as 30 seconds and 60 seconds were 46.8% and 56.5%,respectively, of the amount formed by incubation for 600 seconds (defined as 100%) , but they were 59.4% and 78.1% ,respectively, with 0.96 pmol and 73.4% and 82.9%,respectively,with 1.92 pmol.

### Example 7

In this example, shown is the sensitivity of the assay in which HBsAg was measured using a high concentration of DNP-biotin-anti-HBaAg Fab' on the basis of the result of Example 5, a high concentration of ALP-anti-HBsAg Fab' on the basis of the result of Example 6, large surface areas in accordance with Japanese Patent Application No. 2001-140417 of the magnetic beads on which monoclonal anti-DNP IgG of high ability had been insolubilized in accordance with Japanese Patent Application No. 2000-400853 and of the magnetic beads on which streptavidin had been insolubilized and a method for measurement of the luminescence intensity of luminescent substrate solution for ALP incubated with and subsequently separated from the magnetic beads on which streptavidin had been insolubilized in accordence with Japanese Patent Application No. 2001-189419.

### (Materials and methods)

Materials and methods used are the same as those described in Examples 1 to 3 and 5 except those described below.
. Formation of the immune complex
   In accordance with the method of Example 5, 2.5 pmol of DNP-biotin'anti-HBsAg Fab' and 0.05 IU of HBsAg were incubated for one minute.
. Binding of the immune complex to the magnetic beads on which anti-DNP IgG had been insolubilized
   In accordance with the method of Example 5, the immune complex described above was incubated with 0.5 mg of the magnetic beads on which anti-DNP IgG had been insolubilized for about 1.5 minutes (including 0.5 minute for separation and accumulation of the magnetic beads).
. Binding of ALP-anti-HBsAg Fab' to the magnetic beads on which anti-DNP IgG had been insolubilized
   In accordance with the method of Example 5, the magnetic beads which anti-DNP IgG had been insolubilized on followed by binding of the immune complex described above were incubated with 0.48 pmol of ALP-anti-HBsAg Fab' for about 1 minute (including 20 seconds for separation and accumulation of the magnetic beads ).
. Elution of the immune complex from the magnetic beads on which the anti-DNP IgG had been insolubilized
   In accordance with the method of Example 2, the magnetic beads which anti-DNP IgG had been insolubilized on followed by binding of the immune complex were incubated with 100 µL of 3 mM DNP-Lys solution at room temperature for 1 minute (including 0.5 minute for separation and accumulation of the magnetic beads ), and the supernatant obtained by removing the magnetic beads was used as the eluate.
. Binding of the immune complex to the magnetic beads on which streptavidin had been insolubilized
   In accordance with the method of Example 2, the eluate described above and 0.25 mg of the magnetic beads on which streptavidin had been insolubilized were incubated at room temperature for 1 minute (including 0.5 minute for separation and accumulation of the magnetic beads) and the magnetic beads were washed with 400 µL of washing solution for ALP three times.
. Luminescent assay of ALP activity
   ALP activity bound to the magnetic beads which streptavidin had been insolubilized on followed by binding of the immune complex was measured in the same manner as in Example 3.

### (Results)

The results of Example 7 and those of Comparative Examples 1 and 2 described above are collectively shown in Table 10.The assay of HBsAg by the present invention was performed as follows. DNP-biotin-anti-HBsAg Fab^{'} was allowed to react with HBsAg for one minute. The immune complex of the both was trapped on the first solid phase within 1.5 minutes. By 1 minute incubation with ALP-anti-HBsAg Fab', the immune complex comprising the three was formed on the first solid phase. The immune complex of the three was eluted within 1 minute and transferred to the second solid phase within 1 minute. The luminescence intensity of luminescent substrate solution for ALP was measured after separation from the second solid phase. The sensitivity of this assay by the present invention was 8.2-fold higher than that of the known sandwich method (Comparative Example 1) in which HBsAg was trapped on a microplate for 30 minutes and allowed to react with ALP-anti-HBsAg Fab' for 30 minutes and 6.7- to 13.8-fold higher than that of the known sandwich method (Comparative Example 2) in which HBsAg was trapped on ferrite particles for 10 minutes and allowed to react with ALP-labeled anti-HBsAg antibody (mouse) for 10 minutes.

Thus, the method of the present invention using specifically binding substances at high concentrations, solid phases with large surface areas, monoclonal anti-DNP antibody and measurement of the luminescence intensity of luminescent substrate solution for ALP after separation from solid phase was shown to provide a far more rapid and far more sensitive assay than the known sandwich methods.

Advantages of the present invention shown by Examples 5 to 7 and Comparative Examples 1 and 2

In an assay method in which the complex of a substance to be assayed (analyte) and its specifically binding substance(s) (including modified or labeled ones) is formed on a solid phase (the first solid phase), then transferred to another solid phase (the second solid phase) and finally the complex on the second solid phase is measured for measuring the analyte, use of high concentrations of the specifically binding substance(s) shortened the time required for formation of the complex and provided a rapid and highly sensitive assay. Further, this was combined with shortening of the incubation time for the solid phases with the analyte or the complex by use of large-surface areas, as compared with the volume of the reaction solution containing the analyte or the complex,of the first solid phase or/and the second solid phase incubated with the reaction solution, with shortening of the time required for elution of the complex from the first solid by use of monoclonal antihapten antibody with high ability or/and with measuring the luminescence intensity of the reagent solution for luminescence assay after separation from the second phase to provide a rapid and highly-sensitive assay.

### Example 8

In this example, methods for preparation of monoclonal anti-2,4-dinitrophenyl group antibodies are specifically shown.

### fit Preparation of immunogen

2,4-Dinitrophenylated keyhole limpet hemocyanin (DNP-KLH) (LSL Co., Ltd., Japan) (1mg/mL in terms of KLH) was prepared using a phosphate-buffered physiological saline (pH 7.0) (hereinafter, referred to as PBS) and used as immunogen.

### (2) Animal to be immunized

Female BALB/c mice of inbred strain aged 5 to 8 weeks were raised in a breeding chamber (23 ± 1°C; humidity: 70%) using standard pellets with freely accessible water.

### (3) Method of immunization

Antigen prepared in the above (1), i.e. DNP-KLH was diluted with PBS to 100 µg/0.5 mL and emulsified with the same volume (0.5 mL) of Freund's incomplete adjuvant (Pierce Chemical Company, Rockford, IL). This antigen emulsified (200µ g/mouse) was injected into the peritoneal cavity of five female BALB/c mice aged 5 weeks. Further, every three weeks, 2.0 µg per mouse of the above antigen (100 µ g/mL) prepared with RIBI adjuvant (Adjuvant MPL + TDH Emulsion) (Ribi Immuno Chem Research Inc., Hammilton, MT) were injected three times and the antibody titer of the mice was measured. Three weeks later,DNP-KLH(100 µ g/mL)prepared with PBS was injected into the tail vein of mice with a high antibody titer as a final immunization.

### (4) Cell fusion

Spleen was excised from the BALB/c mice 3 days after the final immunization. The spleen cells were suspended in an EMEM medium, washed with the same medium four times and counted to obtain 5.9 × 10⁸ spleen cells. In cell fusion, a cell line (P3-X63-Ag8.653; hereinafter, referred to as "X63 cells") derived from myeloma of BALB/c mouse resistant to 2-amino-6-oxy-8-azapurine (8-azaguanine) was used as a parent cell line.

The X63 cells were subcultured in an RPMI-1640 medium containing 20µ g/mL 8-azaguanine and 10% heat-inactivated fetal calf serum (hereinafter, referred to as FCS). The X63 cells at logarithmic phase were further cultured in RPMI-1640 medium containing 10% of FCS but devoid of 8-azaguanine for 3 days before cell fusion. The X63 cells at logarithmic phase were washed with RPMI-1640 medium three times and counted to obtain 7 x 10⁷ living cells.

To RPMI-1640 medium, polyethylene glycol 4000 was added to make a concentration of 50 w/v % followed by mixing the spleen cells and X63 cells described above in a ratio of 10:1 for cell fusion according to a known method (Keller and Milstein, *Nature,* volume 256, pages 495-497, 1975 and *European Journal of Immunology,* volume 6, pages 511-519, 1976).

Subsequently, the fused cells were suspended in HAT selection medium (RPMI-1640 medium containing 1 x 10⁻⁴ M hypoxanthine, 4 x 10⁻⁷ M aminopterin, 1.6 × 10⁻⁵ M thymidine and 10% of FCS) to obtain a cell suspension of 2.0 x 10⁶ per ml. Then 50 µL of the cell suspention were planted in each well of a 96-well micro titer plate and incubated in a CO₂ incubator under the atmosphere of 37°C, 95% humidity and 8% CO₂. On the first and the second days after the start of the incubation, one drop of the HAT medium was added to each well, then on the seventh and the ninth days after the start of the incubation, two drops of the HAT medium were added to each well and incubation was further continued. About 10 days to two weeks later, the supernatant of growing hybridoma cells producing the desired anti-DNP monoclonal antibody was tested by the following screening methods.

### (5) Screening

Anti-DNP monoclonal antibody in the culture supernatant from the above hybridoma cells was selected by the reaction to 2,4-dinitrophenylated bovine serum albumin (hereinafter, referred to as DNP-BSA) (LSL Co., Ltd., Japan) immobilized on microplate and εN-2,4-dinitrophenyl-L-lysine (hereinafter, referred to as DNP-Lys).

50 µL each of 1 µ g/mL DNP-BSA as an antigen solution were added to the microplate wells and allowed to be adsorbed overnight. The wells were washed with a phosphate buffer containing 0.05% of Tween 20 (hereinafter, referred to as a washing solution) three times and blocked with a phosphate buffer containing 0.05% of BSA to prepare a plate on which DNP-BSA antigen had been insolubilized.

The culture supernatant of hybridoma cells described above was added to the microplate on which DNP-BSA had been insolubilized followed by the addition of horseradish peroxidase (hereinafter, referred to as HRP) labeled anti-mouse immunoglobulin antibody (derived from goat) for reaction at room temperature for 30 minutes. Subsequently, the plate was washed three times and allowed to react with a substrate solution (containing 2 mg/mL of o-phenylenediamine and 4 mM H₂O₂) at room temperature for 5 minutes. The reaction was stopped with 2N sulfuric acid and the absorbance was measured using an ELISA plate at main wavelength of 492 nm and sub wavelength of 690 nm.

Further, in order to check the eluting ability of monoclonal antibody from the immune complex formed on the plate on which DNP-BSA antigen had been insolubilized, the following screening was carried out. Namely, the culture supernatant of hybridoma cells obtained hereinabove was added to the plate on which DNP-BSA had been insolubilized followed by addition of HRP-labeled anti-mouse immunoglobulin antibody (derived from goat) and allowed to react at room temperature for 30 minutes. After this reaction, it was washed with a washing solution three times and a DNP-Lys solution was added and allowed to react at room temperature for 5 minutes. After this reaction, the plate was washed three times and allowed to react with a substrate solution (containing 2 mg/mL of o-phenylenediamine and 4 mM H₂O₂) at room temperature for 5 minutes. The reaction was stopped by 2N sulfuric acid and the absorbance was measured using an ELISA plate at the main wavelength of 492 nm and the sub-wavelength of 690 nm.

Hybridoma cells producing an antibody having a high ability of elution from the immune complex by the addition of DNP-Lys were selected.

### (6) Establishment of monoclonal antibody-producing hybridoma cell line

The hybridoma cell obtained by the screening in the above (5) was cloned by means of a limiting dilution method. As a result, 8 clones of a hybridoma cell line producing an antibody having a high eluting ability from the immune complex by the addition of DNP-Lys were selected. One clone of the hybridoma line was taken as an established cell line and deposited at International Patent Organism Depositary National Institute of Advanced Industrial Science and Technology as the international deposition number FERM BP-8341 (the deposition number FERM P-18079).

### (7) Identification of mouse immunoglobulin subclass

Mouse immunoglobulin subclasses of 8 monoclonal antibodies produced by each hybridoma clone obtained as a single clone by the cloning described above were identified by MonoAb Typing Kit (Zymed Laboratories, Inc., South San Francisco, CA).

### Example 9

In this example, shown is the time course of eluting the immune complex bound to the magnetic beads on which anti-DNP antibody had been insolubilized.

### (Materials and methods)

Materials and methods used are the same as those in Examples 1 and 2 except those described below.
. Anti-DNP polyclonal antibody (Seikagaku Corporation, Tokyo, Japan)
. Anti-DNP monoclonal antibody, DNP-649
. Anti-DNP monoclonal antibody, DNP-1321
. Anti-DNP monoclonal antibody, DNP-1402 (PERM P-18079)
. Anti-DNP monoclonal antibody, DNP-1623
. Anti-DNP monoclonal antibody, DNP-1753
   Anti-DNP monoclonal antibodies were purified using Protein A.
. DNP-biotin-anti-HBsAg Fab'
   Anti-HBsAg Fab'-740 was substituted for anti-HBsAg Fab'-649 used in Example 1.
. Magnetic beads on which anti-DNP antibody had been insolubilized
   Each of the anti-DNP antibodies was insolubilized on magnetic beads in the same manner as in Example 1.
. Formation of the immune complex
   To each of two culture tubes of 12 × 75 mm were added 90 µL of 4.8 pmol/mL ALP-anti-HBsAg Fab' and 90 µL of 100 pmol/mL DNP-biotin-anti-HBsAg Fab' followed by mixing. To one of them were added 900 µL of 5 IU/mL HBsAg as a positive sample, while 900 µL of TEA buffer as a negative sample were added to the other followed by mixing. The mixture was allowed to stand at room temperature for 10 minutes to prepare an immune complex reaction solution.
. Binding of the immune complex to the magnetic beads on which anti-DNP antibody had been insolubilized
   Meanwhile, 900 µL of 1% suspension of the magnetic beads on which anti-DNP IgG had been insolubilized were added to each of two culture tubes of 12 x 75 mm. Each of them was subjected to magnetic separation and the supernatant was removed by suction.
   To one of the culture tubes were added 1,080 µL of the above positive immune complex reaction solution, and to the other, 1,080 µL of the above negative immune complex reaction solution, followed by mixing. The mixture was allowed to stand at room temperature for 10 minutes. The supernatant was removed.
   After that, the magnetic beads were washed with 3.6 mL of washing solution for ALP twice. Further, 3.6 mL of washing solution for ALP were added and stirred to suspend the particles and 400 µL each of the suspension were added to eight PS tubes of 7 x 50 mm. The tubes were subjected to magnetic separation and the supernatants were removed by suction.
. Elution of the immune complex
   To each tube containing the magnetic beads on which anti-DNP antibody had been insolubilized as mentioned above were added 100 µL of 3mM DNP-Lys followed by mixing and the mixture was allowed to stand for various periods and subjected to magnetic separation for 20 seconds. The supernatant in each tube was removed by suction and the magenetic beads were washed with 0.4 mL of washing solution for ALP three times.
. Fluorometric assay of ALP activity
   ALP activity bound to the magnetic beads after elution and washing described above was measured by fluorometry in the same manner as in Example 1.

### (Results)

The results of Example 9 are shown in Table 11.

With monoclonal anti-DNP antibodies, the elution rates of the immune complex 40 seconds, 1 minute and 2 minutes after addtion of 3mM DNP-Lys were 86.1∼89.8%, 87.0~91.0% and 88.5∼92.5% ,respectively, while they were 26.9% , 31.2% and 40.5% ,respectively, with polyclonal anti-DNP antibody.

### Effects of the present invention shown by Examples 8 and 9

By using the anti-DNP monoclonal antibodies in accordance with the present invention, the immune complexes containing analytes can be eluted from the solid phase more rapidly and more completely, providing more rapid and more sensitive assays of analytes.

### Example 10

In this example, effect of washing is shown in relation to the number of the times for washing by using microfine magnetic particles having large surface areas as the first solid phase according to Japanese Patent Application 2001-140417 and high concentrations of DNP-biotin-anti-HBsAg Fab' and ALP-anti-HBsAg Fab' according to Japanese Patent Application 2001-170186.

### (Materials and methods)

Materials and methods used are the same as those in Example 1 except those described below.
. Step for formation of the immune complex
   DNP-biotin-anti-HBsAg Fab' (1 pmol) and 0.48 pmol of ALP-anti-HBsAg Fab' were incubated in 120 µL of TEA buffer at 37°C for 6 minutes.
. Step for binding of the immune complex to the magnetic beads
   The above solution of 120 µL and 0.5 mg of the magnetic beads which anti-DNP IgG had been insolubilized on and washed with TEA buffer were incubated at room temperature for 1 minute (including 20 seconds for separation and accumulation of the magnetic beads) and the magnetic beads were washed with 0.4 mL of washing solution for ALP once to thrice. The washing was carried out by combining the following two different methods: washing the magnetic beads dispersed in washing solution (dispersed washing) and washing the magnetic beads accumulated without dispersion(accumulated washing). Namely, dispersed washing was carried out once to thrice and, alternatively, one accumulated washing was carried out after one or two dispersed washings or no washing.
. Fluorometric assay of ALP activity
   ALP activity bound to the washed magnetic beads described above was measured in the same manner as in Example 5.

### (Results)

The results of Example 10 are shown in Table 12.

Even when 0.5 mg of the magnetic beads on which anti-DNP IgG had been insolubilized as the first solid phase having as large a surface area as 26 cm² and ALP-anti-HBsAg Fab' having as high a concentration as 4 pmol/mL were incubated in 120 µL of the reaction solution, the ALP activity remaining on the first solid phase (hereinafter, excluding the activity which was non-specifically bound to the solid phase and was not removable by washing) after washing the magnetic beads dispersed in washing solution (hereinafter, referred to as dispersed washing)once was 1/6,702 of the ALP activity used, i.e. 7.16 x 10⁻¹⁷ mol. Accordingly, the ALP activities remaining on the second solid phases subjected to one dispersed washing and on the second solid phase subjected to one washing of the magnetic beads accumulated without dispersion in washing solution (hereinafter, referred to as accumulated washing) after incubation with the eluate separated from the first solid phase which had been dispersed in the eluent and subjected to one dispersed washing were 1/6,702 (1.07 x 10⁻²⁰ mol) and 1/465 (1.54 x 10⁻¹⁹ mol),respectively, of the ALP activity remaining on the first solid phase, fulfilling the condition for an highly sensitive assay. This result showing that a highly sensitive assay is possible even with the first solid phase having as large a surface area as 26 cm² and the ALP-labeled antibody of as high a concentration as 4 pmol/mL indicates that highly sensitive assays are possible using solid phases having smaller surface areas and ALP-labeled antibody of lower concentrations. The ALP activity remaining on the first solid phase subjected to two or three dispersed washings is 1/568,926 or less of the ALP activity used and 8.44 × 10⁻¹⁹ mol or less. The ALP activities remaining on the second solid phases subjected to one dispersed washing, to one accumulated washing and to neither washing (the eluate playing a role as a washing solution) after two or three dispersed washings of the first solid phase are 1/6,702 or less (1.26 × 10^{·22} mol or less), 1/465 or less (1.81 x 10^{·21} mol or less) and 1/40 or less (2.11 x 10^{·20} mol or less), respectively, of the ALP activity remaining on the first solid phase. In addition, the ALP activities remaining on the first solid phase subjected to one dispersed washing and one subsequent accumulated washing was 1/36,213 or less (1.33 x 10⁻¹⁷ mol or less ) of the ALP activity used. The ALP activities remaining on the second solid phases subjected to one dispersed washing, to one accumulated washing and to neither washing after one dispersed washing and one accumulated washing of the first solid phase were 1/6,702 (1.98 × 10⁻²¹ mol), 1/465(2.85 × 10-²⁰ mol) and 1/40 (3.31 x 10⁻¹⁹ mol), respectively, of the ALP activity remaining on the first solid phase. Thus, in any of the cases, a highly sensitive assay was shown to be possible even with the solid phases having larger surface areas and the labeled antibodies having higher concentrations. The ALP activities remaining on the second solid phases subjected to one dispersed washing, to one accumulated washing and to neither washing after two dispersed washings and one accumulated washing of the first solid phase are less than the corresponding activities after two dispersed washings of the first solid phase described above, being apparently capable of providing a more sensitive assay with higher reproducibility and higher precision.

### Example 11

In this example, even on reducing the number of times for washing the solid phase, no significant change is observed in the nonspecific signal, ensuring a highly sensitive assay.

### (Materials and methods)

Materials and methods used are the same as those in Example 3 except those described below.
. Step for formation of the immune complex
   DNP-biotin-anti-HBsAg Fab' and ALP-anti-HBsAg Fab' were incubated in the presence and absence of 0.5 IU of HBsAg at 37°C for 10 minutes.
. Biding of the immune complex to the magenetic beads on which anti-DNP IgG had been insolubilized (first solid phase)
   The first solid phase was incubated with the incubated reaction solution described above and then washed once to thrice.
. Binding of the immune complex to the magnetic beads on which streptavidin had been insolubilized (second solid phase)
   The second solid phase (0.5 mg) was incubated with the eluate containing the immune complex and then washed once or twice.
. Luminescent assay of ALP activity
   The second solid phase washed was incubated with luminescent substrate solution for ALP used in Example 3 at room temperature for 10 minutes and subjected to magnetic separation. The whole supernatant was transferred to a microplate used in Example 1 and its luminescence intensity was measured in the same manner as in Comparative Example 1.

### (Results)

The luminescence intensities as nonspecific signals, that is, those in the absence of HBsAg among the results of Example 11 are shown in Table 13.

On washing the first solid phase once to thrice and the second solid phase once or twice, no significant difference was observed in the nonspecific signals by the number of times for washing. In addition, the luminescence intensities as the nonspecific signals shown in Table 13 were not greatly different from those obtained by subjecting the second solid phase before use to luminescent assay in the same manner as in this example. No significant difference was observed in the specific signal in the presence of HBsAg, either. This indicates that a highly sensitive assay is possible even using a solid phase in fine particles having a weigh of 0.5mg, that is, as large a surface area as 26 cm² when both the first and second solid phases are washed once and that a further improvement of the sensitivity is possible by lowering ALP activity shown by the second solid phase itself.

Advantages of the present invention shown by Examples 10 and 11

In an assay method in which the complex(es) of a substance to be assayed(analyte) and its specifically binding substance(s) (including modified or labeled ones) is formed on a solid phase (first solid phase), and then transferred to another solid phase (second solid phase) and the complex on the second solid phase is measured to measure the analyte,the reduction of the number of times for washing of both the first and second solid phases down to one each made possible the followings : to shorten the time required for washing the solid phases, consequently to provide a rapid and highly sensitive assay,at the same time, to decrease the total volumes of washing and waste solutions and consequently to reduce the sizes of vessels for the both and automatic assay apparatus. Further, this was combined with shortening the incubation time to as short a time as one minute of the first or/and second solid phases with the reaction solution containing the analyte or the complex by using the first or/and second solid phases with larger surface areas relative to the volume of the reaction solution, shortening the time required for formation of the complex(es) by raising the concentration of the specifically binding substance(s), shortening the time required for elution of the complex(es) from the first solid phase to as short a time as one minute by using monoclonal antihapten antibody with high ability or /and measuring the luminescence intensity of the reagent solution for luminescent assay after separation from the second phase, making it possible to provide a more rapid and more highly sensitive assay.

### Example 12

In this example, it is shown possible to measure the luminescence intensity of a reagent solution for luminescence assay of alkaline phosphatase (hereinafter, referred to as ALP) after transfer to an untreated microplate well following contact with the enzyme insolublized on a microplate well.

### (Materials and methods)

Materials and methods used are the same as those in Examples 1 to 3 except those described below.
. Microplate
   Fluoro Nunc™ Module Plate, C8 Maxisorp white, Nunc A/S, DK-4000 Roskilde, Denmark
. ELSIA F washing solution, International Reagents Corporation, Kobe, Japan
. Biotinyl-ALP
   Bionityl alkaline phosphatase (Pierce Chemical Company, Rockford, IL)
. Microplate well on which streptavidin had been insolubilized
   100 µL each of 30 µg/mL biotinyl-bovine serum albumin dissolved in phosphate buffer were added to microplate wells and allowed to stand at 4°C overnight. The wells were washed with ELSIA F washing solution three times and subsequently 100 µL each of 30 µg/mL streptavidin dissolved in phosphate buffer were added. After allowing to stand at 4°C overnight, the wells were washed with ELSIA F washing solution three times. After addition of 300 µL each of blocking solution, the wells were stored at 4°C.
. Binding of biotinyl ALP to the microplate well on which streptavidin had been insolubilized
   100 µL each of TEA buffer or 1.9 fmol/mL biotinyl ALP dissolved in TEA buffer were added to the microplate wells on which streptavidin had been insolubilized and the microplates were shaken at room temperature for 40 minutes. Subsequently, the wells were washed with 300 µL of washing solution for ALP three times.
. Luminescent assay of ALP activity
   To the above microplate wells which biotinyl ALP was bound to and washed were added 200 µL of luminescent substrate solution for ALP used in Example 3 and allowed to stand at 37°C for 10 minutes. The substrate solution was transferred to an untreated microplate well to measure luminescent intensity in the same manner as in Comparative Example 1.

### (Results)

The results of Example 12 are shown in Table 14.

The luminescence intensity of luminescent substrate solution for ALP measured even after transfer to an untreated microplate well following incubation in a microplate well which streptavidin had been insolubilized on and biotinyl-ALP had been bound to was 67% of that measured without transfer and, in addition, did not decrease rapidly. The luminescence intensity of the microplate well, which streptavidin had been insolubilized on followed by binding of biotinyl-ALP, after removal of luminescent substrate solution for ALP increased with the lapse of time, and the luminescence intensity of 200 µL of luminescent substrate solution for ALP newly added to the microplate well increased with the lapse of time and, after 10 minutes, up to that observed with neither removal nor transfer of the reagent solution. This indicates that biotinyl ALP bound to the microplate on which streptavidin had been insolubilized was not eluted into luminescent substrate solution for ALP. Therefore, the luminescence intensity of luminescent substrate solution for ALP measured after the transfer was not due to the activity of alkaline phosphatase eluted from the microplate well into the substrate solution.

### Example 13

This example shows the effect of the present invention on luminescent assay of ALP activity bound to magnetic beads.

### (Materials and methods)

Materials and methods used are the same as those in Examples 1 to 3 and 12 except those described below.
. Binding of biotinyl ALP to the magnetic beads on which streptavidin had been insolubilized
   Biotinyl ALP (5 × 10⁻¹⁷ mol/mL) dissolved in TEA buffer (100 µL) and 0.5 mg of the magnetic beads on which streptavidin had been insolubilized were mixed and stirred at room temperature for 15 minutes. Subsequently, the magnetic beads after magnetic separation were washed with 400 µL of washing solution for ALP three times.
. Luminescent assay of ALP activity
   To the above magnetic beads which streptavidin had been insolubilized on followed by binding of biotinyl ALP were added 200 µL of the luminescent substrate solution for ALP used in Example 3 and incubated at room temperature for 5 minutes. The magnetic beads were subjected to magnetic separation for 30 seconds and removed and the luminescent intensity of the supernatant was measured in the same manner as in Example 3. Luminescence intensity before removal of the magnetic beads was also measured.

### (Results)

The results of Example 13 are shown in Table 15.

Very low values were observed on measuring the luminescence intensity after mixing ALP bound to the magnetic beads and the luminescent substrate solution for ALP. However, on measuring the luminescence intensity of luminescent substrate solution for ALP after separation and removal of the magnetic beads, 5.8-fold higher value was obtained. Thus, interference of measurement of luminescence intensity due to quenching by the magnetic beads could be eliminated by removal of the magnetic beads, showing the effect of the present invention.

### Effects of the present invention shown by Examples 12 and 13

In an assay method for an analyte by measuring the amount of luminescent substance produced in the presence of label substance bound to the solid phase for assay of the analyte, it became possible to measure luminescence intensity without limitation by types, forms, sizes, amounts of solid phase, methods for insolubilization, etc. in the following manner. The luminescence intensity of reagent solution for luminescent assay was measured after incubation with and subsequent separation from the solid phase which a label substance producing a luminescent substance in the reagent solution had been bound to. This made it possible to provide a highly sensitive assay of the analyte.

### Example 14

In this example, using whole blood and serum collected at the same time from a healthy person, the sensitivity of a two-step sandwich assay method for HBsAg is shown to be significantly lowered with whole blood as compared with serum.

### (Materials and methods)

Materials and methods used are the same as those in Examples 1 to 3 and Comparative Example 1 except those described below.
. Whole blood
   Blood was collected in tube for plasma from a healthy person A and was used as whole blood. Serum of A was negative to HBsAg.
. Serum
   Serum was separated from blood collected in a tube for serum from A.
. Magnetic beads on which anti-HBsAg IgG had been insolubilized
   Monoclonal anti-HBsAg IgG (No. 649; International Reagents Corporation, Kobe, Japan) was insolubilized on magnetic beads through covalent bond according to the directions of JSR Corporation and stored in blocking solution at 4°C.
. Measurement of HBsAg
   10 µL of 1% suspension in TEA buffer of the magnetic beads on which anti-HBsAg IgG had been insolubilized, 240 µL of TEA buffer containing 0.15M NaCl,5 mg/mL casein, 10% bovine serum and 0.5% mouse serum and 100 µL of whole blood, serum, whole blood containing 0.5 IU of HBsAg or serum containing 0.5 IU of HBsAg were added to a test tube of 7 x 50 mm and incubated at 37°C for 10 minutes with stirring. The reaction solution was separated and removed from the magnetic beads using a magnet and the magnetic beads were washed once with 400 µL of washing solution for ALP. Then 250 µL of TEA buffer containing 0.45 pmol of ALP-anti-HBsAg Fab' were added to the magnetic beads and incubated at 37°C for 10 minutes with stirring. The magnetic beads were separated from the reaction solution using a magnet to remove the reaction solution and washed with 0.4 mL of washing solution for ALP three times.
. Luminescent assay of ALP activity
   To the washed magnetic beads were added 200 µL of luminescent substrate solution for ALP used in Example 3 followed by stirring, and the whole suspension of the magnetic beads was transferred to a microplate and incubated at 37°C for 5 minutes. The luminescence intensity was measured in the same manner as in Comparative Example 1.

### Example 15

The steps used were entirely the same as in Example 14 except the assay of ALP activity bound to the magnetic beads.

Fluorescent substrate solution for ALP (100 µL) was added to the magnetic beads washed in the last step of Example 14, stirred and incubated at 37°C for 30 minutes. Subsequently, 50 µL of 0.36 M K₂HPO₄ were added as a solution for stopping the reaction, followed by magnetic separation of the magnetic beads and fluorescence intensity of the whole supernatant was measured in the same manner as in Example 1.

### Example 16

In this example, both sensitivities for HbsAg in whole blood and serum are shown to be identical in the assay using the steps A,B and C according to the present invention.

### (Materials and methods)

Materials and methods used are the same as those in Examples 1 to 3 except those described below.
. Tubes on which streptavidin had been insolubilized
   Phosphate buffer (150 µl) containing 30 µg/ml biotinylated bovine serum albumin prepared by a known method (E. Ishikawa, Ultrasensitive and Rapid Enzyme Immunoassay, pages 177-302; aforementioned) was added to an F-tube (International Reagents Corporation), allowed to stand at 4°C overnight and the tube was washed with 500 µl of phosphate buffer three times. Then, 150 µl of phosphate buffer containing 30 µg/ml streptavidin were added to the tube and allowed to stand at 4°C overnight. The tube was washed with 500 µl of phosphate buffer three times and was stored at 4°C after addition of 500 µl of blocking solution.
. Formation of the immune complex
   50 µL of whole blood, serum, whole blood containing 0.05 IU of HBsAg or serum containing 0.05 IU of HBsAg, 50 µL of TEA buffer containing 0.15 M NaCl, 5 mg/mL casein, 10% bovine serum and 0.5% mouse serum and 20 µL of TEA buffer containing 1 pmol of DNP-biotin-anti-HBsAg Fab' and 0.48 pmol of ALP-anti-HBsAg Fab' were incubated at room temperature for 10 minutes.
. Binding of the immune complex to the magnetic beads on which anti-DNP IgG had been insolubilized.
   The reaction solution containing the immune complex described above was incubated for 1 minute with 0.5mg of the magnetic beads on which anti-DNP IgG had been insolubilized (first solid phase). The first solid phase was separated with a magnet and washed with 400 µL of washing solution for ALP three times.
. Elution of the immune complex from the first solid phase
   The first solid phase washed as described above was incubated at room temperature for 1 minute with 200 µL of TEA buffer containing 3 mM εN-2, 4-dinitrophenyl-L-lysine and was separated with a magnet.
. Binding of the immune complex to the second solid phase
   The supernatant described above was incubated at 37°C for 5 minutes with the F-tube (second solid phase) on which streptavidin had been insolubilized and the second solid phase was washed with 1mL of washing solution for ALP three times.
. Luminescent assay of ALP activity
   The second solid phase washed as described above was incubated at 37°C for 5 minutes with 200 µL of luminescent substrate solution for ALP used in Example 3 and then luminescence intensity was measured in the same manner as in Example 3.

### (Results)

The results of Examples 14 to 16 are collectively shown in Table 16.

In Examples 14 and 15 using a known sandwich method, nonspecific signals with whole blood were remarkably higher than those with serum and the sensitivity for the assay of HBsAg was makedly lowered with whole blood. In constrast, in Example 16 using an assay method according to the present invention, there was no big difference between whole blood and serum in terms of both nonspecific and specific signals and similar sensitivities were achieved with whole blood and serum.

### Effect of the present invention shown by Example 16

A factor(s) to interfere with the assay using whole blood as sample can be eliminated by the method of the present invention, in which the complex of an analyte and its specifically-binding substance(s) (including modified or labeled ones) is formed and fixed on a solid phase (first solid phase), then the complex is eluted, transferred and fixed on another solid phase (second solid phase) and finally the complex on the second solid phase is measured for measuring the analyte. Further, it is possible to eliminate a factor(s) interfering with the assay using whole blood and to provide a rapid and highly sensitive assay method for analytes contained in or mixed with whole blood by incorporating into this method the following methods: a method of shortening the time for fixation (trapping) of an anayte or/and the complex on the solid phase using a solid phase having large surface areas, a method of shortening the time for binding a specifically-binding substance(s) to the corresponding analyte using its high concentrations, a method of shortening the time for elution of the complex from the first solid phase using monoclonal antihapten antibody with high ability, a method of shortening the time for washing, the solid phases by reducing the times for washing the solid phase or/and a method of measuring the luminescence intensity of reagent solution for luminescent assay after separation from the solid phase incubated.

**Table 1**

| Relation between Diameter, Surface Area and Volume of Sphere | | | | | | |
|---|---|---|---|---|---|---|
| Diameter (µm) | Surface Area per Sphere (mm²) | Volume per Sphere (mm³) | Number | Volume (mm³) | Number | Volume (mm³) |
| | | | per 1000 mm² Surface Area | | per 5000 mm² Surface Area | |
| 1 | 3.14x10⁻⁶ | 5.23×10⁻¹⁰ | 3.18×10⁸ | 0.17 | 1.59 x 10⁹ | 0.83 |
| 10 | 3.14x10⁴ | 5.23x10⁻⁷ | 3.18x10⁶ | 1.7 | 1.59 x 10⁷ | 8.3 |
| 100 | 3.14 x 10-² | 5.23 x 10⁻⁴ | 3.18 × 10⁴ | 17 | 1.59 × 10⁵ | 83 |
| 1000 | 3.14 | 5.23×10⁻¹ | 3.18 × 10² | 170 | 1.59 × 10³ | 833 |
| 1 mm² = 0.01 cm² | | | | | | |

**Table 2**

| Methods and Times for Washing of Solid Phase and Washing Effect | | | | | | | |
|---|---|---|---|---|---|---|---|
| First Solid Phase | | Second Solid Phase | | | Object for Measuring Concentration or Amount of A | Concentration of A | Amount of A |
| Number of times for washing | Removal of washing solution | Removal of eluate | Number of times for washing | Removal of washing solution | | | |
| - | No | No | - | No | Reaction solution | 1 | 1 |
| 1 | No | No | - | No | washing solution for first solid phase | 1/1.5x10² | - |
| 1 | Yes | No | - | No | First solid phase | - | 1/6×10³ |
| 1 | Yes | No | - | No | Eluate | 1/6x10³ | - |
| 1 | Yes | Yes | - | No | Second solid phase | - | 1/2.4×10⁵ |
| 1 | Yes | Yes | 1 | No | washing solution for second solid phase | 1/9×10⁵ | - |
| 1 | Yes | Yes | 1 | Yes | Second solid phase | - | 1/3.6×10⁷ |
| 2 | No | No | - | No | Second washing solution for first solid phase | 1/2.25x10⁴ | |
| 2 | Yes | No | - | No | First solid phase | - | 1/9x10⁵ |
| 2 | Yes | No | - | No | Eluate | 1/9x10⁵ | - |
| 2 | Yes | Yes | - | No | Second solid phase | - | 1/3.6x10⁷ |
| 2 | Yes | Yes | 1 | No | washing solution for second solid phase | 1/1.35×10⁸ | - |
| 2 | Yes | Yes | 1 | Yes | Second solid phase | - | 1/5.4×10⁹ |

The concentration of labeled specifically-binding substance (A) in washing solution and eluate and the amount of A remaining on the first and the second solid phases were calculated theoretically assuming the volumes of reaction solution containing A incubated with the first solid phase and eluate (eluent)to be both 100 µL, and the volume of washing solution to be 400 µL.

**Table 3**

| Amount of Immune Complex Trapped vs. Surface Area of Magnetic Beads on which Anti-DNP IgG had been Insolubilized (Example 1) | | | | | | | |
|---|---|---|---|---|---|---|---|
| Magnetic Beads | | Fluorescence Intensity of ALP Activity Specifically Bound to Magnetic Beads | | | | | |
| Weight (mg) | Surface Area (cm²) | Incubation Time (minutes) | | | | | |
| | | 1 | | 2 | | 10 | |
| 0.1 | 5.2 | 1,795 | | 2,610 | | 3,774 | |
| | | (43.5) | [47.6] | (63.3) | [69.2] | (91.5) | [100] |
| 0.2 | 10.4 | 2,953 | | 3,754 | | 4,112 | |
| | | (71.6) | [71.8] | (91.1) | [91.3] | (99.7) | [100] |
| 0.5 | 26 | 3,985 | | 4,087 | | 4,095 | |
| | | (96.7) | [97.3] | (99.1) | (99.8] | (99.3) | [100] |
| 1 | 52 | 4,050 | | 4,100 | | 4,123 | |
| | | (98.2) | [98.2] | (99.4) | [99.4] | (100) | [100] |

The fluorescence intensity of alkaline phosphatase (APL) activity specifically bound was calculated by subtracting that bound in the absence of HBsAg from that bound in the presence of 0.05 IU of HBsAg.

The values in ( ) indicate percentages of the value obtained by incubation with 1 mg of the magnetic beads for 10 minutes which was defined as 100%.

The values in [ ] indicate percentages of the values obtained by incubation with indicated amounts of the magnetic beads for 10 minutes, which were defined as 100%.

**Table 4**

| Amount of Immune Complex Trapped vs. Surface Area of Magnetic Beads on which Streptavidin had been Insolubilized (Example 2) | | | | | | | |
|---|---|---|---|---|---|---|---|
| Magnetic Beads | | Fluorescence Intensity of ALP Activity Specifically Bound to Magnetic Beads | | | | | |
| Weight (mg) | Surface Area (cm²) | Incubation Time (minutes) | | | | | |
| | | 1 | | 2 | | 10 | |
| 0.1 | 5.2 | 1,472 | | 2,101 | | 3,025 | |
| | | (45.3) | [48.7] | (64.7) | [69.5] | (93.2) | [100] |
| 0.2 | 10.4 | 2,392 | | 3,059 | | 3,274 | |
| | | (73.7) | [73.1] | (94.2) | [93.4] | (100.9) | [100] |
| 0.5 | 26 | 3,152 | | 3,298 | | 3,246 | |
| | | (97.1) | [97.1] | (101.6) | [101.6] | (100) | [100] |

The fluorescence intensity of alkaline phosphates (APL) activity specifically bound was calculated by subtracting that bound in the absence of HbsAg from that bound in the presence of 0.05 IU of HBsAg.

The values in ( ) indicate percentages of the value obtained by incubation with 0.5 mg of the magnetic beads for 10 minutes which was defined as 100%.

The values in [ ] indicate percentages of the values obtained by incubation with indicated amounts of the magnetic beads for 10 minutes which were defined as 100%.

**Table 5**

| Amount, Surface Area and Reaction Time of Magnetic Beads and Sensitivity of Assay (Example 3) | | | | | | | | |
|---|---|---|---|---|---|---|---|---|
| First Solid Phase | | | Second Solid Phase | | | Luminescence Intensity of ALP Activity Bound to Second Solid Phase | | |
| Amount (mg) | Surface Area (cm²) | Incubation Time (min) | Amount (mg) | Surface Area (cm²) | Incubation Time (min) | Amount of HBsAg Added (lU) | | P-N/N |
| | | | | | | 0 (N) | 0.05 (P) | |
| 0.1 | 5.2 | 1 | 0.1 | 5.2 | 1 | 262 | 35,948 | 136 |
| 0.5 | 26 | 1 | 0.25 | 13 | 1 | 298 | 135,698 | 454 |

**Table 6**

| Comparison of Sensitivities for HBs Ag by the Assay Method of the Present Invention and Known Sandwich Methods | | | | |
|---|---|---|---|---|
| Examples and Comparative Examples | Assay Method | Luminescence Intensity | | P-N/N |
| | | Amount of HBsAg Added (lU) | | |
| | | 0(N) | 0.05 (P) | |
| Example 3 | Present Invention (Magnetic Beads) | 298 | 135,698 | 454 |
| Comparative Example 1 | Known Sandwich Method (Microplates) | 0.0387 | 1,275 | 32 |
| Comparative Example 2 | Known Sandwich Method | (A) 0.0016 | 0.0323 | 19 |
| | (Magnetic Beads) | (B) 287 | 11,400 | 39 |
| A: The assay was performed manually using the solid phase and reagents of Fuji Rebio Lumipulse II HBsAg and the luminescence intensity was measured in microplates in the same manner as in Comparative Example 1. | | | | |
| B: The assay was performed using the solid phase, reagents and automatic assay apparatus of Fuji Rebio Lumipulse II HBsAg. | | | | |

**Table 7**

| Comparison of Trapping Rate of HBsAg on Solid Phase on which Anti-HBsAg Antibody had been Insolubilized by Known Sandwich Method and the Method of the Present Invention | | | |
|---|---|---|---|
| Method | Incubation Time with Solid Phase on which Anti-HBsAg Antibody had been Insolubilized (min) | | Percentage of Luminescence Intensity or Fluorescence Intensity of Activity Specifically Bound to Solid Phase |
| | HBsAg | ALP- Anti-HBsAg | % |
| Known Sandwich Method (Comparative Example 3) | 2 | 10 | 29.6 |
| | 3 | 10 | 42.7 |
| | 10 | 10 | 95.2 |
| | 20 | 10 | 100 |
| Method of Present Invention (Example 4) | 1 | 1 | 55.8 |
| | 2 | 1 | 71.1 |
| | 5 | 1 | 94.4 |
| | 10 | 1 | 100 |

**Table 8**

| Concentration of DNP-Biotin-Anti-HBsAg Fab' vs. Amount of DNP-Biotin-Anti-HBsAg Fab'-HBsAg Complex Formed (Example 5) | | | |
|---|---|---|---|
| DNP-Biotin-Anti-HBsAg Fab' (pmol/test) | Fluorescence Intensity of ALP Activity Specifically Bound to First Solid Phase | | |
| | Incubation Time of DNP-Biotin-Anti-HBsAg Fab' with HBsAg [0.5 ID/test] | | |
| | 5 sec | 1 min | 10 min |
| | Incubation Time with Magnetic Beads on which Anti-DNP IgG had been Insolubilized | | |
| | 1 min | 1 min | 1 min |
| 0.5 | 2,377 [40.5] | 3,641 [62.0] | 5,875 [100] |
| | (30.6) | (46.9) | (75.7) |
| 2.5 | 5,077 [70.3] | 7,484 [103.6] | 7,222 [100] |
| | (65.4) | (96.4) | (93.0) |
| 5 | 5,541 [71.4] | 7,421 [95.6] | 7,762 [100] |
| | (71.4) | (95.6) | (100) |

The incubation time with the magnetic beads (1 mg) on which anti-DNP IgG had been insolubilized was the time from the start of mixing with them until immediately before the start of mixing with washing solution after their accumulation.

The values in ( ) indicate percentages of the value obtained using 5 pmol, 10 minutes with HBsAg and 1 minute with the magnetic beads which was defined as 100%.

The value in [ ] indicate percentages of the values obtained with indicated amounts in pmol, 10 minute with HBsAg and 1 minute with the magnetic beads, which were defined as 100%.

**Table 9**

| Concentration ofALP-Anti-HBsAg Fab' vs. Amount of DNP-Biotin-Anti-HBsAg Fab'-HbsAg-ALP-Anti-HBsAg Fab' Complex Formed on the First Solid Phase (Example 6) | | | |
|---|---|---|---|
| ALP-Anti-HBsAg Fab' pmol/test | Fluorescence Intensity of ALP Activity Specifically Bound to First Solid Phase | | |
| | Incubation Time of ALP-Anti-HBsAg Fab' with First Solid Phase | | |
| | 30 se | 60 sec | 600 sec |
| 0.48 | 4,334(46.8] | 5,238 [56.5] | 9,266 [100] |
| | (40.3) | (48.8) | (86.3) |
| 0.96 | 6,015 [59.4] | 7,910 [78.1] | 10,128 [100] |
| | (56.0) | (73.6) | (94.3) |
| 1.92 | 7,889 [73.4) | 8,910 [82.9] | 10,743 [100] |
| | (73.4) | (82.9) | (100) |

The incubation time of ALP-anti-HBsAg Fab' with the magnetic beads which Anti-DNP IgG had been insolubilized on followed by binding of DNP-biotin-anti-HBsAg Fab' and HbsAg is the time from the start of mixing of the magnetic beads with the reaction solution until immediately before the start of mixing them with washing solution after their accumulation.

The values in ( ) indicate percentages of the value obtained with 1.92 pmol and 10 minutes which was defined as 100%.

The value in [ ] indicate percentages of the values obtained with indicated amounts in pmol and 10 minutes which were defined as 100%.

**Table 10**

| Comparison of Sensitivities for HBsAg by the Assay Method of the Present Invention and Known Sandwich Methods | | | | |
|---|---|---|---|---|
| Examples and Comparative Examples | Assay Method | Luminescence Intensity | | P-N/N |
| | | Amount of HBsAg Added (IU) | | |
| | | 0 (N) | 0.05 (P) | |
| Example 7 | Present Invention (Magnetic Beads) | 286 | 75,214 | 262 |
| Comparative Example 1 | Known Sandwich Method (Microplates) | 0.0387 | 1,275 | 32 |
| Comparative Example 2 | Known Sandwich Method (Magnetic Beads) | (A) 0.0016 | 0.0323 | 19 |
| | | (B) 287 | 11,400 | 39 |
| A and B are the same as those in Table 6. | | | | |

**Table 11**

| Time Course Of Elution Rate of Immune Complex from the Magnetic Beads on which Anti-DNP Antibody had been Insolubilized (Example 9) | | | | |
|---|---|---|---|---|
| Monoclonal Anti-DNP Antibody (No.) | DNP-Lys solution (mM) | Elution Rate of Immune Complex (%) | | |
| | | Incubation Time for Elution (min.) (including Time for Magnetic Separation) | | |
| | | 0.67 | 1 | 2 |
| 649 | 3 | 87.6 | 90.7 | 92.0 |
| 1,321 | 3 | 88.0 | 90.3 | 92.3 |
| 1,402 | 3 | 89.5 | 90.6 | 92.5 |
| 1,623 | 3 | 86.1 | 87.0 | 88.5 |
| 1,753 | 3 | 89.8 | 91.0 | 92.1 |
| Polyclonal | 3 | 26.9 | 31.2 | 40.5 |

**Table 12**

| Washing Efficiency of the First Solid Phase of Magnetic Beads (Example 10) | | | | |
|---|---|---|---|---|
| Number of Times for Washing | | ALP Activity Remaining on First Solid Phase | | |
| Dispersed Washing | Accumulated Washing | Fluorescence Intensity (Value Observed) | Fluorescence Intensity of Activity not Bound to Solid Phase (Value observed minus 628) | Comparison of Activity not Bound to Solid Phase with That Used |
| 0 | 1 | 47,155 | 46,527 | 1/465 |
| 1 | 0 | 3,854 | 3,226 | 1/6,702 |
| 1 | 1 | 1,225 | 597 | 1/36,213 |
| 2 | 0 | 666 | 38 | 1/568,926 |
| 3 | 0 | 628 | 0 | |
| The fluorescence intensity (628) observed after three dispersed washings was assumed to be that of ALP activity (non-specifically) bound to the solid phase and not removable. | | | | |

**Table 13**

| Number of Times for Non-Competitive Washing Solid Phase and Nonspecific Signal in the Complex-Transfer Assay Method (Example 11) | | |
|---|---|---|
| Number of Times for Washing | | Luminescence Intensity as Nonspecific Signal |
| First Solid Phase | Second Solid Phase | |
| 1 | 1 | 0.005 |
| 1 | 2 | 0.0055 |
| 2 | 1 | 0.0055 |
| 2 | 2 | 0.0057 |
| 3 | 1 | 0.0047 |
| 3 | 2 | 0.0054 |

**Table 14**

| Luminescence Intensity Obtained by Measurement after Separation of Reagent Solution for Luminescent Assay from Solid Phase (Example 12) | | | | |
|---|---|---|---|---|
| Time after Separation (sec) | Luminescence Intensity | | | |
| | No Separation (A) | Separated Solid Phase (B) | Separated Solution (C) | (B) + (C) |
| 10 | 86(100) | 15(17) | 58(67) | 73(85) |
| 29 | 86(100) | 20(23) | 55(64) | 75(87) |
| 46 | 86(100) | 23(27) | 52(60) | 75(87) |
| 65 | 85(99) | 27(31) | 48(56) | 75(87) |
| 82 | 85(99) | 29(34) | 45(52) | 74(86) |
| 101 | 86(100) | 32(37) | 42(49) | 74(86) |
| 119 | 85(99) | 34(40) | 39(45) | 73(85) |
| The numbers in parentheses indicate percentages. | | | | |

**Table 15**

| Elimination of Interference with Luminescent Assay by Magnetic Beads (Example 13) | |
|---|---|
| Removal of Magnetic Beads | Percentage of Luminescence Intensity of ALP Activity bound to Magnetic Beads |
| Not Removed | 100 |
| Removed | 575 |

**Table 16**

| Comparison of Sensitivities for HBsAg Added to Serum and Whole Blood by Known Two-Step Sandwich Method and the Non-Competitive Complex-Transfer Assay Method . | | | | | | |
|---|---|---|---|---|---|---|
| Example | Sample | Luminescence or Fluorescence Intensity | | | | |
| | | Amount of HBsAg Added (IU) | Addition of HBsAg | | | |
| | | | Not Added (N) | Added (P) | (P)-(N) | [(P)-(N)]/(N) |
| Example 14 (Luminescence) | Serum | 0.5 | 0.0076 | 0.3829 | 0.3753 | 49 |
| | Whole Blood | 0.5 | 0.9102 | 1.2435 | 0.3333 | 0.37 |
| Example 15 (Fluorescence) | Serum | 0.5 | 122 | 1,269 | 1,147 | 9.4 |
| | Whole Blood | 0.5 | 27.920 | 28,558 | 638 | 0.023 |
| Example 16 (Fluorescence) | Serum | 0.05 | 770 | 82,090 | 81,320 | 106 |
| | Whole Blood | 0.05 | 807 | 85,210 | 84,403 | 105 |

## Claims

1. A non-competitive complex-transfer assay method for measuring analytes comprising the following three steps A, B and C using at least one of the methods described in the following (1) to (7).
Step A: a step in which the complex(es)of an analyte( a substance to be assayed) with a substance(s) (including modified or labeled ones) which binds specifically to the analyte (hereinafter described as specifically binding substance) is formed on a solid phase (a first solid phase).
Step B: a step in which the complex(es) formed on the first solid phase in the step A is eluted and transferred to another solid phase (a second solid phase).
Step C: a step in which the complex(es) transferred to the second solid phase in the step B is measured.
(1) a method using, in at least one of the steps A and B described above, at least one solid phase in a form of small or fine particles which has a substance(s) insolubilized for trapping an analyte or the complex(es) of the analyte with its specifically binding substance(s) (including labeled or modified ones) and has such a large surface area that the amount of the analyte or the complex(es) trapped on the solid phase by 3 minute incubation of the both is 60% or more of the maximum amount of the analyte or the complex(es) trapped on the solid phase by a sufficiently long incubation of the both which is defined as 100%.
(2) a method using at least one concentration of a specifically binding substance (including labeled or modified ones )which is so high that the amount of the complex(es) formed of the specifically binding substance with the corresponding analyte by 3 minute incubation of the both is 60% or more of the maximum amount of the complex(es) formed by a sufficiently long incubation of the both which is defined as 100%.
(3) a method using at least one bond between the complex(es) and the first solid phase which ensures that the amount of the complex(es) eluted from the first solid phase by 3 minute incubation in the eluent is 60% or more of the total amount of the complex(es) fixed on the first solid phase before elution which is defined as 100%.
(4) a method in which washing of the first solid phase after the formation of the complex(es) of an analyte with its specifically binding substance(s)(including modified or labeled ones) in the step A and before starting elution of the complex(es) in the step B(hereinafter, referred to as washing of the first solid phase) is carried out once and washing of the second solid phase after trapping the complex(es) in the step B and before starting the step C (hereinafter, referred to as washing of the second solid phase)is carried out once; or a method using two washings of the first solid phase and one washing of the second solid phase or no washing of the second solid phase.
(5) a method using a common first solid phase and a common second solid phase for different analytes regardless of time and place of the assay.
(6) a method in which luminescence intensity of reagent solution for luminescent assay incubated with a substance(s) bound to a solid phase which produces a luminescent substance(s) in the presence of the reagent solution or regulates its production is measured after its separation from the solid phase.
(7) a method in which the complex(es) is formed on the first solid phase in the presence of whole blood which has been diluted 100-fold or less in a part or all of the step A.

2. The non-competitive complex-transfer assay method described in the claim 1 using a solid phase(s) in a form of small or fine particles described in the claim 1(1) which are magnetic or magnetizable ones.

3. The non-competitive complex-transfer assay method described in the claim 1 or 2 using a solid phase(s) in a form of small or fine particles described in the claim 1(1) which have a mean diameter of 0.2 to 200 µm or equivalent ones.

4. The non-competitive complex-transfer assay method described in the claim 1 or 2, using a solid phase(s) in a form of small or fine particles described in the claim 1(1) which have a mean diameter of 0.5 to 20 µm or equivalent ones.

5. The non-competitive complex-transfer assay method described in any of the claims 1 to 4,using a solid phase(s) in a form of small or fine particles described in the claim 1(1) having a surface area expressed in cm², the value of which is 50- to 1,000-fold larger than the value of the volume expressed in mL of the reaction solution containing an analyte or the complex(es) to be incubated with the solid phase or equivalent ones.

6. The non-competitive complex-transfer assay method described in any of the claims 1 to 5, using the incubation time of an analyte or the complex(es) with a solid phase for trapping them described in the claim 1(1) which is 10 seconds to 3 minutes.

7. The non-competitive complex-transfer assay method described in any of the claims 1 to 6, using at least one concentration of specifically binding substances described in the claim 1(2) which is 2 to 100 pmol/mL.

8. The non-competitive complex-transfer assay method described in any of the claims 1 to 7, using the incubation time of an analyte and its specifically binding substances described in the claim 1(2) which is 10 seconds to 3 minutes.

9. The non-competitive complex-transfer assay method described in any of the claims 1 to 8, using the elution time of the complex(es) from the first solid phase described in the claim 1(3) which is 10 seconds to 3 minutes.

10. The non-competitive complex-transfer assay method described in any of the claims 1 to 9, using a bond of antihapten antibody(ies) with a hapten (including a hapten derivative(s)) for at least one bond between the complex(es) and the solid phase described in the claim 1(3) and using the hapten (including a hapten derivative(s)) for elution of the complex(es) from the solid phase.

11. The non-competitive complex-transfer assay method described in the claim 10, using an anti-dinitrophenyl group antibody(ies) as the antihapten antibody and a compound containing dinitrophenyl group(s) as the hapten (including a hapten derivative(s)).

12. A monoclonal anti-dinitrophenyl group antibody, retaining such a property that the bond between the antibody and the compound containing dinitrophenyl group(s) described in the claim 11 is dissociated to an extent of 60% or more within 3 minutes after addition of 1 to 3 mM dinitrophenyl-lysine.

13. The monoclonal antibody described in the claim 12 produced by hybridoma cells of the international deposition number FERM BP-8341 (the deposition number FERM P-18079).

14. A hybridoma cell of the international deposition number FERM BP-8341 (the deposition number FERM P-18079).

15. The non-competitive complex-transfer assay method described in the claim 10 or 11 using the monoclonal antibody described in the claim 12 or 13.

16. The non-competitive complex-transfer assay method described in any of the claims 1 to 11 and 15 using the total time for conducting the steps A and B excluding the time for washing the solid phases which is 2 minutes to 10 minutes in combination with at least the three methods described in the claims 1(1), 1(2) and 1(3).

17. The non-competitive complex-transfer assay method described in any of the claims 1 to 11, 15 and 16 using two or three washings of the first solid phase in a form of small or fine particles described in the claim (1) and one or two washings of the second solid phase in a form of small or fine particles described in the claim 1(1) on condition that the solid phase in a form of small or fine particles is washed by dispersing the particles in washing solution(hereinafter, referred to as dispersed washing).

18. The non-competitive complex-transfer assay method described in the claim 17 substituting, for one of the two or three washings of the first solid phase, one washing in which accumulated particles are washed without dispersion (hereinafter, referred to as accumulated washing).

19. The non-competitive complex-transfer assay method described in the claim 17 or 18 substituting one accumulated washing for one of the one or two washings of the second solid phase.

20. The non-competitive complex-transfer assay method described in any of the claims 1 to 11 and 15 to 19, using a common first solid phase for different analytes described in the claim 1(5) on which monoclonal antihapten antibody(ies) has been insolubilized.

21. The non-competitive complex-transfer assay method described in the claim 20 using a monoclonal anti-2,4-dinitrophenyl group antibody or the antibody described in the claim 12 or 13 as monoclonal antihapten antibody.

22. The non-competitive complex-transfer assay method described in any of the claims 1 to 11 and 15 to 21 using a common second solid phase for different analytes described in the claim 1(5) on which monoclonal antihapten antibody(ies) which is different from that insolubilized on the first solid phase has been insolubilized.

23. The non-competitive complex-transfer assay method described in any of the claims 1 to 11 and 15 to 22 using a common second solid phase for different analytes described in the claim 1(5) on which avidin or streptavidin has been insolubilized.

24. The non-competitive complex-transfer assay method described in any of the claims 1 to 11 and 15 to 23 using hydrolase(s) as a substance(s) which is bound to a solid phase and produces a luminescent substance(s) in the presence of the reagent solution for luminescent assay described in the claim 1(6).

25. The non-competitive complex-transfer assay method described in the claim 24 using alkaline phosphatase as hydrolase.

26. The non-competitive complex-transfer assay method described in the claim 24 using β -D-galactosidase as hydrolase.

27. The non-competitive complex-transfer assay method described in any of the claims 24 to 26 using a reagent solution for luminescent assay containing a dioxetane derivative(s) as a luminescent substrate.

28. An assay kit containing at least one solid phase and/or reagent for carrying out the non-competitive complex-transfer assay method described in any of the claims 1 to 11 and 15 to 27.

29. An assay system containing at least one solid, phase and/or reagent for carrying out the non-competitive complex-transfer assay method described in any of the claims 1 to 11 and 15 to 27.
